(19) Europäisches Patentamt
European Patent Office
Office européen des brevets

(11) Publication number : 0 370 320 B1

(12) EUROPEAN PATENT SPECIFICATION

(45) Date of publication of patent specification : 30.09.92 Bulletin 92/40

(51) Int. Cl.$^5$ : **A61K 31/49**, A61K 31/47, A61K 31/395, A61K 31/55

(21) Application number : 89120782.1

(22) Date of filing : 09.11.89

# (54) Lactamimides in the treatment of drug-resistant protozoal infections.

(30) Priority : 10.11.88 US 269858

(43) Date of publication of application : 30.05.90 Bulletin 90/22

(45) Publication of the grant of the patent : 30.09.92 Bulletin 92/40

(84) Designated Contracting States : AT BE CH DE ES FR GB GR IT LI LU NL SE

(56) References cited : WO-A-80/03802

(56) References cited : BIOLOGICAL ABSTRACTS, vol. 88, no. 8, 1989, page 808, abstract no. 88338, Biological Abstracts Inc., Philadelphia, PA, US;M.G. PALFREYMAN et al.: "Lactamimdes: A novel chemical class of calcium antagonists with diltiazem-like properties", & BIOCHEMPHARMACOL 38(15): 2459-2466

(73) Proprietor : MERRELL DOW PHARMACEUTICALS INC.
2110 East Galbraith Road
Cincinnati Ohio 45215-6300 (US)

(72) Inventor : Bitonti, Alan J.
7854 Carraway Ct.
Mainville Ohio 45039 (US)

(74) Representative : Vossius & Partner
Siebertstrasse 4 P.O. Box 86 07 67
W-8000 München 86 (DE)

EP 0 370 320 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to the use of certain lactamimide derivatives in the treatment or prevention of drug-resistant malaria and in the treatment or prevention of other drug-resistant protozoal infections.

Malaria remains a significant health threat to humans despite massive international attempts to eradicate the disease. Over 200 milion people are said to have malaria and over one million deaths per year are associated with malaria in Africa alone. In many of the endemic areas, local supply of food is quite limited, a problem which is greatly aggravated by the presence of protozoal infections in cattle and other farm animals.

Malaria is a disease of warm blooded animals caused by infection with a parasite of the genus Plasmodium. Four species, P. *vivax*, P. *falciparum*, P. *malariae*, and P. *ovale*, are known to infect humans. The parasite is transmitted to humans by the bite of *Anopheles* mosquitoes. Subsequent to mosquito bite, the parasite rapidly invades the blood cells of the victim and after an incubation period, generally lasting about 10 to 14 days, symptoms, consisting of chills, fever, headache, muscle pains, splenomegaly, and anemia, appear. This incubation period may be prolonged for many weeks and onset can be quite insidious. Red blood cells are at first altered and later destroyed by the infection. After an acute episode, the victim may be reinfected by the parasite which persists in the liver.

Drug therapy utilizing quinine, chloroquine, amodiaquine, primaquine, and other agents has been the mainstay of therapy against malaria and diseases caused by infection by other protozoa such as various species of Leishmaniasis and Trypanosomiasis. However, drug-resistant strains of protozoa have developed and in some cases strains are resistant to many or all of the current therapeutic agents. In particular, P. *falciparum* malaria is quite prone to exhibit single and even multiple drug-resistance. While new agents are continually developed and introduced, resistance to such new agents also quickly develops. For example mefloquine-resistant malaria was reported even before mefloquine licensure was completed. There is, thus, an urgent need for antimalarial and antiprotozoal agents which can be used in the treatment of drug-resistant malaria and other protozoal diseases.

Applicants have discovered that certain previously known lactamimide derivatives when admininstered in conjunction with standard antimalarial agents, are highly effective in treating or preventing drug-resistant malarial and other drug-resistant protozoal infections.

Applicants have found that certain lactamimide derivatives of formula I

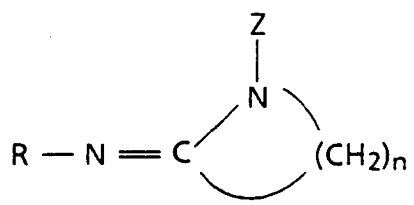

Formula I

wherein

Z is hydrogen or lower alkyl of from 1 to 4 carbon atoms;

n is an integer of from 3 to 16;

R is

A) a straight or branched alkyl group of from 8 to 15 carbon atoms; or a lower alkoxyalkyl group wherein the alkyl moiety has from 8 to 15 carbon atoms and the alkoxy moiety has from 1 to 4 carbon atoms;

B) the group phenylalkyl wherein the alkyl moiety has from 1 to 6 carbon atoms and wherein the phenyl moiety is unsubstituted or substituted in which case the substituents may be attached at the ortho, meta or para-position of the phenyl ring and are chlorine, fluorine, bromine, lower alkyl having from 1 to 4 carbon atoms, lower alkoxy having from 1 to 4 carbon atoms, hydroxy or methylenedioxy;

C) the group

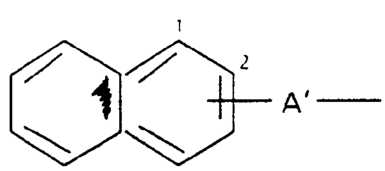

wherein A' is a straight or branched alkylene group of from 1 to 6 carbon atoms or benzyl and is

attached to either the 1- or 2-position of the naphthalene ring, which is either unsubstituted or is mono- or di-substituted with a chlorine, fluorine, bromine, trifluoromethyl, a straight or branched alkyl group of from 1 to 12 carbon atoms, an alkoxy group of from 1 to 3 carbon atoms or $NO_2$;

D) the group 1- or 2-adamantyl or 1- or 2-norbornyl;

E) the group ortho, meta or para-biphenylyl;

F) the group 9-fluorenyl which is unsubstituted or substituted in which case the substituents may be attached to any one of the four available carbon atoms of each aromatic ring and are chlorine, bromine, fluorine, a lower alkyl group having from 1 to 4 carbon atoms, a lower alkoxy group having from 1 to 4 carbon atoms or $NO_2$;

G) dibenzocycloheptenyl;

H) the group

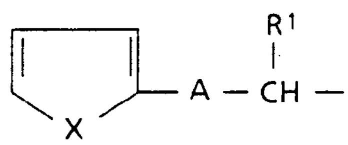

wherein X is oxygen or sulfur, A is a bond or an alkylene chain of from 1 to 3 carbon atoms, and $R^1$ is hydrogen, a straight or branched lower alkyl group having from 1 to 4 carbon atoms, a straight or branched lower alkenyl group having from 3 to 6 carbon atoms, cycloalkyl of from 3 to 6 carbon atoms or phenyl;

I) the group

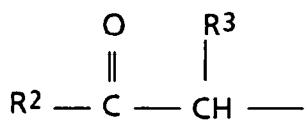

wherein $R^2$ is phenyl or a cycloalkyl group having from 3 to 6 carbon atoms, and $R^3$ is hydrogen or methyl;

J) the group

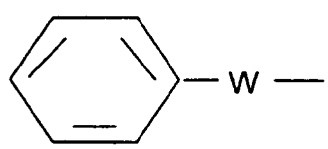

wherein W is a straight or branched alkylene chain having from 2 to 6 carbon atoms which is substituted with one phenyl group on any of the 6 carbon atoms with the proviso that the carbon atom adjacent to the exocyclic nitrogen atom must have at least one hydrogen attached to it;

K) the group

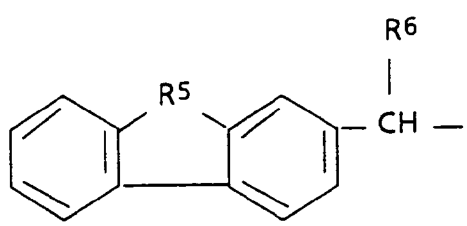

wherein $R^5$ is oxygen, sulfur, $-CH_2-$, $-CH_2CH_2-$ or $-CH=CH-$, and $R^6$ is hydrogen or a lower alkyl group of from 1 to 4 carbon atoms;

L) the group

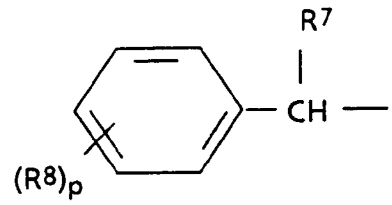

wherein $R^7$ is cycloalkyl of from 3 to 5 carbon atoms, $R^8$ is hydrogen, lower alkoxy of from 1 to 4 carbon atoms or lower alkyl of from 1 to 4 carbon atoms, and p is the integer 1 or 2;

M) the group

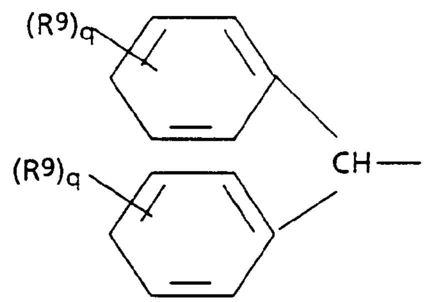

wherein $R^9$ is hydrogen, a straight or branched lower alkyl group of from 1 to 4 carbon atoms, chlorine, fluorine, bromine, $CF_3$, $SCF_3$, $OCF_3$, phenyl, phenoxy or a lower alkoxy group of from 1 to 4 carbon atoms and q is an integer of from 1 to 3;

N) the group

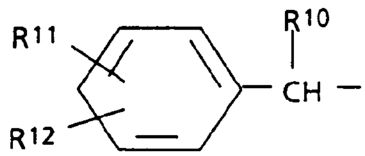

wherein $R^{10}$ is a lower alkyl group of from 1 to 4 carbon atoms, $R^{11}$ is hydrogen or lower alkyl of from 1 to 4 carbon atoms, $R^{12}$ is an alkyl group having from 8 to 14 carbon atoms, an alkoxy group having from 8 to 14 carbon atoms, a cycloalkyl group having from 5 to 14 carbon atoms, phenyl, phenoxy, phenylalkyl wherein the alkyl moiety has from 1 to 4 carbon atoms, phenylalkoxy wherein the alkoxy moiety has from 2 to 4 carbon atoms, 2,2-diphenylvinyl or fluoren-9-ylidene;

O) the group

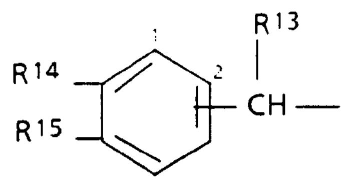

wherein the phenyl moiety is attached to the

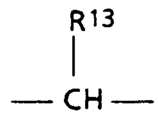

moiety through the 1- or 2-position of the phenyl ring, $R^{13}$ is a lower alkyl group of from 1 to 4 carbon atoms and $R^{14}$ and $R^{15}$ taken together are $(-CH_2-)_3$, $-CH_2CH_2C(CH_3)_2-$, $-(CH_2)_4-$ or $-C(CH_3)_2CH_2CH_2C(CH_3)_2-$;

P) the group

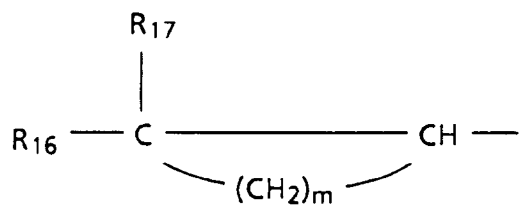

wherein $R_{16}$ is thienyl, cycloalkyl of from 5 to 7 carbon atoms, or is benzyl, phenyl, or substituted phenyl or benzyl, in which case the substitutents on the substituted phenyl are selected from fluorine, chlorine, bromine, iodine, lower alkyl of from 1 to 4 carbon atoms, and lower alkoxy of from 1 to 4 carbon atoms; Z is hydrogen or hydroxy; and m is an integer of from 3 to 6;

with the proviso that when R is the group

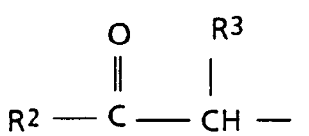 ,

Z is hydrogen;

and pharmaceutically acceptable acid addition salts thereof,

or a pharmaceutically acceptable salt thereof when administered conjunctively with an antiprotozoal agent are useful as pharmaceutical compositions in the treatment of individuals suffering from and prevention of drug-resistant malaria and other drug-resistant protozoal infections.

It is apparent from the foregoing general Formula I that all of the compounds utilized in the instantly claimed invention contain a lactamimide ring having the structure

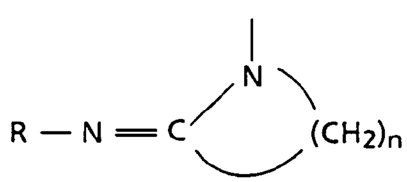

wherein n is an integer of from 3 to 16. Thus, the heterocyclic ring of the lactamimide moiety can be, for example, pyrrolidine, piperidine, hexahydroazepine, octahydroazocine, octahydroazonine, azacyclodecane, azacycloundecane, azacyclododecane or azacyclotridecane each of which is attached to the exocyclic nitrogen of the lactamimide moiety through the 2-position.

The term "dibenzocycloheptenyl" as used herein means both 5H-dibenzo[a,d]cycloheptenyl and 5H-dibenzo[a,c]cycloheptenyl wherein the open valency is on any of the saturated carbon atoms of the cycloheptane ring. That is, the term dibenzocycloheptenyl means

5H-dibenzo[a,d]cyclohepten-5-yl,

5H-dibenzo[a,d]cyclohepten-10(or 11)-yl,

5H-dibenzo[a,c]cyclohepten-5(or 7)-yl, and

5H-dibenzo[a,c]cyclohepten-6-yl.

It is readily apparent that many of the "R" groups of the compounds of formula I have geometric or optical isomers. Unless specifically indicated, the individual isomers and their mixtures are intended.

For convenience and uniformity the compounds described herein are represented and named as substituted 2-iminoperhydroazacarbocyclics, as represented by general Formula I. It is known that compounds of this type as acid addition salts may also be represented by the tautomeric form illustrated by the following general Formula II:

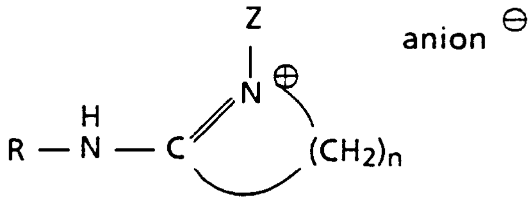

Formula II

This tautomerism has been discussed by R. Kwok and P. Pranc, J. Org. Chem. 32, 740 (1967). Structures of this formula could be named differently. In solution under the used conditions the proportions of each tautomeric form or the delocalization of the charge between the two nitrogen atoms will be dependent upon numerous factors including the nature of the substituents and the pH of the medium. This equilibrium state is conveniently illustrated by the following general Formula III:

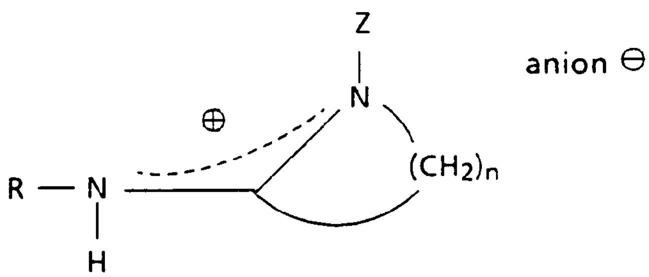

Formula III

The compounds of general Formula I wherein R is other than the group

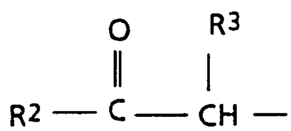

as acid addition salts can exist in either tautomeric form, and it is understood that this invention relates to the use of compounds represented or named in either tautomeric form. In the above general Formulas II and III, the various symbols R, Z and n have the meanings defined in general Formula I. Further, when Z has the meaning hydrogen, other generally known tautomerism can occur.

Compounds of general Formula I wherein R represents

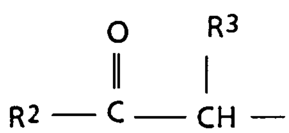

and Z represents hydrogen may exist as a cyclic tautomer of the following general Formula IV.

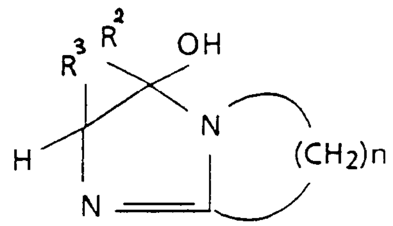

Formula IV

In the above general Formula IV the various symbols n, $R^2$ and $R^3$ have the meanings defined in general Formula I. It is understood that this invention embraces the use of the compounds of general Formula I wherein R is the group

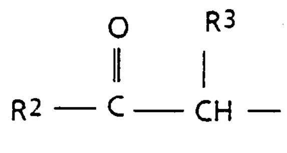

when in the form as represented by general Formula IV. For purposes of convenience the compounds of this type are named herein as the cyclic form.

A preferred embodient of this invention is the use of the compounds described herein wherein n is an integer of from 0 to 16, and within this preferred embodiment the use of the compounds wherein n is the integer 11 is more preferred. Also preferred are those compounds of formula I wherein R is selected from the groups D, E, L, and P. More preferred are those compounds of formula I wherein R is selected from 1- or 2-norbornyl, especially 2-norbornyl, ortho-biphenyl, or a diphenylalkyl group of from 2 to 6 carbon atoms wherein the phenyl groups are substituted on the β or δ carbon atom of the alkyl chain, especially a 2,2-diphenylpentyl or 3,3-diphenylpropyl group as well as those compounds wherein R is a phenyl substituted cycloalkyl group. Especially preferred are those compounds of formula I wherein n is II and wherein R is 2-norbornyl, ortho-biphenylyl,2,2-diphenylpentyl, or a 2-phenylcyclopentyl group.

Illustrative examples of compounds of this invention are:

2-(8-methoxyoctylimino)azacyclotridecane,
2-(12-n-propoxydodecylimino)azacyclododecane,
hexahydro-2-(10-butoxydecylimino)azepine,
2-(11-ethoxyundecylimino)piperidine,
2-(12-n-propoxydodecylimino)pyrrolidine,
2-(benzylimino)pyrrolidine,
2-(p-chlorobenzylimino)piperidine,
hexahydro-2-(3′,4′-methylenedioxybenzylimino)azepine,
2-(p-tolylimino)azacyclodecane,
octahydro-2-(p-anisylimino)azonine,
octahydro-2-(p-n-butoxyphenethylimino)azocine,
hexahydro-2-(o-propylphenethylimino)azepine,
hexahydro-2-(o-tolylimino)azepine,
hexahydro-2-(benzylimino)azepine,
hexahydro-2-[1-(6-dodecyl-1-naphthyl)ethylimino]azepine,
2-(7-trifluoromethyl-2-naphthylmethylimino)octahydroazonine,
hexahydro-2-(1-naphthylmethylimino)azepine,
2-[1-naphthyl)ethylimino]piperidine,
2-(6-bromo-2-methoxy-1-naphthylmethylimino)azacyclotridecane,
2-[1-(5,8-dimethyl-1-naphthyl)ethylimino]hexahydroazepine,
2-[1-(1-naphthyl)ethylimino]octahydroazepine,
hexahydro-2-α-(1-naphthyl)benzyliminoazepine,
2-1-(adamantylimino)azacyclotridecane,
2-(2-adamantyl)hexahydroazepine,
2-(1-adamantylimino)azacycloundecane,
1-methyl-2-(2-adamantylimino)azacyclododecane,
2-(2-adamantylimino)azacyclodecane,

2-(2-norbornylimino)azacyclododecane,
1-methyl-2-(1-norbornylimino)azacyclodecane,
2-(2-norbornylimino)piperidine,
1-ethyl-2-(2-norbornylimino)hexahydroazepine,
2-(2-norbornylimino)pyrrolidine,
2-(1-norbornylimino)azacyclotridecane,
2-(o-biphenylylimino)azacyclotridecane,
2-(m-biphenylylimino)hexahydroazepine,
1-methyl-2-(p-biphenylylimino)piperidine,
2-(o-biphenylylimino)pyrrolidine,
2-(9-fluorenylimino)azacyclotridecane,
2-(9-fluorenylimino)pyrrolidine,
2-(2-nitro-9-fluorenylimino)piperidine,
2-(3-methoxy-9-fluorenylimino)hexahydroazepine,
2-(9-fluorenylimino)octahydroazepine,
2-(3,6-dimethyl-9-fluorenylimino)hexahydroazepine,
1-methyl-2-(9-fluorenylimino)azacyclotridecane,
2-(3-methyl-7-chloro-9-fluorenylimino)azacyclododecane,
2-(9-fluorenylimino)azacycloundecane,
2-(4-bromo-9-fluorenylimino)azacyclodecane,
2-(3-n-propoxy-9-fluorenylimino)pyrrolidine,
2-(2-fluoro-9-fluorenylimino)piperidine,
2-[(10,11-dihydro-5H-dibenzo[a,d]cycloheptan-5-yl)imino]hexahydroazepine,
2-[(10,11-dihydro-5H-dibenzo[a,d]cycloheptan-5-yl)imino]octahydroazocine,
2-[(10,11-dihydro-5H-dibenzo[a,d]cycloheptan-5-yl)imino]octahydroazocine,
2-[(10,11-dihydro-5H-dibenzo[a,d]cycloheptan-5-yl)imino]azacyclotridecane,
2-[(10,11-dihydro-5H-dibenzo[a,d]cycloheptan-5-yl)imino]azacyclododecane,
2-[(10,11-dihydro-5H-dibenzo[a,d]cycloheptan-5-yl)imino]piperidine,
2-[(10,11-dihydro-5H-dibenzo[a,d]cycloheptan-5-yl)imino]pyrrolidine,
2-[(1-benzylcyclopentyl)imino]pyrrolidine,
2-[[α-(2-thienyl)benzyl]imino]azacyclotridecane,
2-[(2-thienyl)imino]hexahydroazepine,
1-methyl-2-(2-thienylimino)hexahydroazepine,
hexahydro-2-[1-(2-thienyl)propylimino]azepine,
2-(furfurylimino)hexahydroazepine,
2-[[α-(2-furyl)benzyl]imino]octahydroazocine,
1-ethyl-2-[[α-(2-furyl)benzyl]imino]octahydroazocine,
2-[[α-(2-thienyl)benzyl]imino]hexahydroazepine,
2-[1-(2-thienyl)propylimino]pyrrolidine,
2-[(α-cyclopropyl-2-thienyl)imino]hexahydroazepine,
2-[β-(2-thienyl)isopropylimino]hexahydroazepine,
2-[2-(1-[2-furyl]pent-4-enyl)imino]azacyclotridecane,
2-[2-(1-[2-furyl]pent-4-enyl)imino]azacyclododecane,
2-[(α-cyclopentyl-2-thienyl)imino]piperidine,
2-[(α-[2-furyl]benzyl)imino]hexahydroazepine,
2-[(3-[4-(2-furyl)-4-cyclopentyl]butylimino)azepine,
2-(-[1-(2-furyl)hex-4-enyl]imino)azacyclotridecane,
2,3,5,6,7,8-hexahydro-2-methyl-2-phenylimidazo[1,2-a]pyridin-3-ol,
2,3,6,7-tetrahydro-2-methyl-3-phenyl-5H-pyrrolo[1,2-a]imidazol-3-ol,
2,3,6,7-tetrahydro-2,2-dimethyl-3-phenyl-5H-pyrrolo[1,2-a]imidazol-3-ol,
2,3,5,6,7,8,9,10-octahydro-2-methyl-3-phenylimidazo-[1,2-a]azocine-3-ol,
2,3,5,6,7,8-hexahydro-3-phenylimidazo[1,2-a]pyridin-3-ol,
3-cyclohexyl-2,3,6,7,8,9-hexahydro-2-methyl-5H-imidazo[1,2-a]azepine-3-ol,
hexahydro-2-[(β-methyl-β-phenylphenethyl)imino]azepine,
2-[(β-ethyl-β-phenylphenethyl)imino]octahydroazocine,
2-[(1,3-diphenylpentyl)imino]octahydroazonine,
2-[(1,3-diphenylpropyl)imino]piperidine,
2-(2,2 diphenylethylimino)hexahydroazepine,

2-(3,3-diphenylpropylimino)hexahydroazepine,
2-(2,3-diphenylpropylimino)azacyclotridecane,
2-[(α-methyl-β-phenylphenethyl)imino]hexahydroazepine,
2-(1,4-diphenylbutylimino)pyrrolidine,
1-methyl-2-(1,4-diphenylbutylimino)azacyclododecane,
2-[(β-phenyl-β-propylphenethyl)imino)azacyclotridecane,
2-[β-isopropyl-β-phenylphenethyl)imino]hexahydroazepine,
2-(α-phenylphenethylimino)pyrrolidine,
2-[(α-methyl-β-phenethylpropyl)imino]octahydroazocine,
2-[(β-phenyl-β-propylphenethyl)imino]octahydroazonine,
1-butyl-2[l-(2-dibenzothienyl)ethylimino]pyrrolidine,
2-[1-(2-fluorenyl)ethylimino]hexahydroazepine,
2-[2-methyl-1-(3-phenanthryl)propylimino]octahydroazocine,
2-[1-(2-dibenzofuranyl)propylimino]piperidine,
2-[1-(4-dibenzothienyl)ethylimino]hexahydroazepine,
2-[1-(2-fluorenyl)pentylimino]octahydroazonine,
2-[1-(9-phenanthryl)ethylimino]hexahydroazepine,
2-[1-(2-phenanthryl)ethylimino]azacyclotridecane,
2-[1-(9,10-dihydro-2-phenanthryl)propylimino]azacyclodecane,
2-[1-(2-dibenzothienyl)ethylimino]-N-methylhexahydroazepine,
2-[1-(2-dibenzothienyl)ethylimino]octahydroazocine,
2-[(2-dibenzofuranyl)ethylimino]octahydroazonine,
2-[1-(3-phenanthryl)ethylimino]azacycloundecane,
2-[(α-cyclopropylbenzyl)imino]piperidine,
2-[(α-cyclopropyl-α-methoxybenzyl)imino]pyrrolidine,
2-[(α-cyclopropyl-p-butylbenzyl)imino]octahydroazepine,
2-[(α-cyclopropyl-tert-butoxybenzyl)imino]hexahydroazepine,
2-[(α-cyclobutylbenzyl)imino]azacyclotridecane,
2-[(α-cyclobutyl-2,4-diethylbenzyl)imino]octahydroazocine,
2-[(α-cyclopropylbenzyl)imino]-1-methylpyrrolidine,
2-[(α-cyclopropyl-2,4-dimethylbenzyl)imino-1-methylhexahydroazepine,
1-ethyl-2-[(α-cyclobutylbenzyl) imino]azacyclodecane,
1-propyl-2-[(α-cyclopentylbenzyl)imino]hexahydroazepine,
2-[(α-cyclobutyl-3,5-dimethoxybenzyl)imino]azacyclododecane,
2-[(α-cyclopentylbenzyl)imino]octahydroazonine,
2-[(α-cyclopentyl-2,4-dipropoxybenzyl)imino]azacyclotridecane,
2-[[α-cyclopentyl-p-ethoxybenzyl)imino]octahydroazocine,
2-[(bis[p-ethylphenyl]methyl)imino]octahydroazocine,
2-[(p-chloro-α-phenylbenzyl)imino]hexahydroazepine,
hexahydro-2-[(α-[m-methylphenyl]-m-isopropylbenzyl)imino]octahydroazonine,
2-[(bis[p-trifluoromethylthiophenyl]methyl)imino]piperidine,
2-[(m-[trifluoromethyl-α-phenyl]benzyl)imino]hexahydroazepine,
2-[(p-chloro-α-[p-chlorophenyl]benzyl)imino]azacyclodecane,
2-[(α-[p-chlorophenyl]-m-trifluoromethylbenzyl)imino]azacycloundecane,
2-[(p-tert-butyl-α-[p-chlorophenyl]benzyl)imino]azacyclododecane,
1-methyl-2-[(α-phenyl-p-trifluoromethoxybenzyl)imino]hexahydroazepine,
2-[(p-bromo-α-phenylbenzyl)imino]azacyclotridecane,
2-[(p-phenoxy-α-phenylbenzyl)imino]azacyclotridecane,
2-[(3,4,5-trimethoxy-α-phenylbenzyl)imino]azacyclododecane,
2-[(3,4-dipropoxy-α-phenylbenzyl) imino]octahydroazepine,
2-[(bis[p-trifluoromethylthiophenyl]methyl)imino]azacyclotridecane,
2-[(diphenylmethyl)imino]-1-methylpiperidine,
2-[(diphenylmethyl)imino]octahydroazocine,
2-[(diphenylmethyl)imino]-N-octahydroazonine,
2-[(m-methoxy-α-phenylbenzyl)imino]hexahydroazepine,
2-[(p-butoxy-α-phenylbenzyl)imino]octahydroazonine,
2-[(diphenylmethyl)imino]azacyclotridecane,
2-[(p-decyl-α-methylbenzyl)imino]pyrrolidine,

1-methyl-2-[α-methyl-p-3,3-diethylpentyl)benzylimino]pyrrolidine,
1-ethyl-2-[p-(dodecyloxy)-α-methylbenzylimino]piperidine,
2-[ methyl-p-(3,7-dimethyloctyloxy)benzylimino]piperidine,
1-propyl-2-[α-butyl-p-cyclohexylbenzylimino]hexahydroazepine,
2-[α-methyl-p-cyclododecylbenzylimino]hexahydroazepine,
2-[2,2-diphenylvinyl)-α-isopropylbenzylimino]azacyclodecane,
2-(α-methyl-p-tridecylbenzylimino)hexahydroazepine,
2-[α,2-dimethyl-4-(3-phenoxypropoxy)benzylimino]octahydroazonine,
2-[α,5-dimethyl-2-(3-phenylethoxy)benzylimino]octahydroazocine,
2-[α,3-dimethyl-4-(3-phenylpropoxy)benzylimino]hexahydroazepine,
2-(p-dodecyloxy-2-methylbenzylimino)hexahydroazepine,
1-methyl-2-[α-methyl-p-phenoxybenzylimino]azacycloundecane,
2-[α-methyl-p-(3-phenyl-1-propen-1-yl)benzylimino]azacyclotridecane,
2-[α-methyl-p-(4-phenylbutoxy)benzylimino]azacyclododecane,
2-[p-(2-phenoxyethoxy-α-methylbenzylimino]azacyclotridecane,
1-[1-(1,2,3,4-tetrahydro-6-naphthyl)ethylimino]hexahydroazepine,
2-[1-(4-indanyl)ethylimino]hexahydroazepine,
2-[1-(1,2,3,4-tetrahydro-2,2,4,4-tetramethyl-6-naphthyl)propylimino]azacyclotridecane,
1-methyl-2-[1-4-indanyl) ethylimino]azacyclododecane,
2-[1-(1,2,3,4-tetrahydro-6-naphthyl)ethylimino]piperidine,
2-[1-(1,1-dimethyl-4-indanyl)ethylimino]azacyclotridecane,
2-[1-(4-indanyl)ethylimino]pyrrolidine,
hexahydro-2-[(trans-2-phenylcyclopentyl)imino]azepine,
2-[2-([p-chlorophenyl]cyclopentyl)imino]hexahydroazepine,
1-butylhexahydro-2-[(cis-2-phenylcyclopentyl)imino]azepine,
hexahydro-2-[2([o-tolyl]cyclohexyl)imino]azepine,
2-[2-([m-anisyl]cyclohexyl) imino]azacyclododecane,
2-[(2-phenylcyclopentyl)imino]azacyclotridecane,
2-[(2-phenylcycloheptyl)imino]azacycloundecane,
2-[(2-cyclohexylcyclopentyl)imino]hexahydroazepine,
hexahydro-2-[(trans-2-phenylcyclopropyl)imino]azepine,
2-(2-phenylcyclopropylimino)azacyclotridecane,
2-[(2-phenylcyclopentyl)imino]piperidine,
octahydro-2-[(2-phenylcyclobutyl)imino]azonine,
2-[(2-[2-thienyl]cyclopentyl)imino]pyrrolidine, and
2-[(2-cyclohexylcyclopentyl)imino]piperidine hydrochloride.

The compounds of this invention can be prepared as described in the prior art, i.e., U.S. patent number 4,061,746.

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intended to apply to any non-toxic organic or inorganic acid addition salts of the base compounds of formula I. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution.

The term "patients" as used herein means those warm-blooded animals, such as cattle, sheep, goats, swine, and primates including humans, manifesting diseases caused by drug-resistant protozoal infections. The most important use of the present invention is its use in the treatment of drug-resistant protozoal infections in humans.

As used herein, the term "protozoal infections" means those protozoal infections of warm-blooded animals by such genera as Plasmodium (including such species as vivax, malariae, ovale, falciparum, knowlesi, berghei, vinckei, chabaudi, gallinaceum, and lophurae), Leishmania (including such species as donovani, tropica, braziliensis, and mexicana), Trypanosoma (including such species as cruzi), Babesiosis, and Theileria species.

The most important use contemplated for the present invention is its use in the treatment of drug-resistant Plasmodium infections, especially malarial infections of drug-resistant strains of P.*falciparum*, in humans.

The term "drug-resistant protozoal infections" means those protozoal infections in which the infecting strain of protozoa is either completely or substantially refractory to therapy with existing antiprotozoal agents or combinations of antiprotozoal agents. The term "drug-resistant malaria" means a malarial infection, particularly of malaria resulting from infection by P.*falcipurum* in humans, which are substantially not responsive to treatment with existing therapeutic agents such as quinine, chloroquine, amodiaquine, primaquine, or mefloquine.

The antiprotozoal agents used in the combination therapy or prophylaxis of this invention include any therapeutic agent used in treating non drug-resistant protozoal infections and those therapeutic agents currently used in treating or preventing drug-resistant protozoal infections such as mefloquine. As used herein, the terms "antiprotozoal agent" and "antimalarial agent" specifically do not include the compounds of formula I. Examples of antiprotozoal agents used in treating or preventing malarial infections are various quinoline derivatives such as quinine, chloroquine, primaquine, sulfadoxine, mefloquine, and pyrimethamine. Examples of antiprotozoal agents used in treating Leishmaniasis infections are various pentavalent antimony compounds such as sodium stibogluconate, amphotericin B, and pentamidine isothionate. Examples of antiprotozoal agents used in treating Trypanosomiasis infections are nifurtimox, suramin, melarsoprol, alpha-difluoromethylornithine (DFMO), and pentamidine. Various salts of these agents may also be employed and combinations of these various agents are routinely utilized.

The term "conjunctive therapy" as used herein contemplates the administration of a formula I compound immediately prior to, concomitantly with, or subsequent to treatment with the antiprotozoal agent or agents. The use of compound I "immediately prior to" or "subsequent to" the antiprotozoal agent can also be designated as a "separate sequential" use of I and the antiprotozoal agent. Applicants contemplate that the lactamimide compounds of this invention may be formulated into a single dosage form together with the antiprotozoal agent; however, such a combination dosage form is not required in order to practice the method of this invention. Typically, treatment of a patient infected with a drug-resistant protozoa, requires doses of the antiprotozoal agent or agents many times the normal dosage, and such therapy is heroic in nature, i.e., in an effort to save the life of the patient, doses of antiprotozoal agents normally regarded as "overdosages" are used and symptomatic relief of overdosage symptoms is tended to on an individual basis. While the conjunctive therapy and prophylaxis of this invention will provide for use of less antiprotozoal agent than would be possible in the absence of the formula I compound, applicants contemplate that the dose of antiprotozoal agent employed in the method of this invention will be essentially that dose which would be employed in the absence of the formula 1 compound when used to treat or prevent a non drug-resistant protozoal infection. Rather than decreasing the dose of antiprotozoal agent required, the method of this invention will provide for treatment or prevention of protozoal infections which would otherwise not be adequately treated in the absence of a formula 1 compound. In fact, it is the very gist of applicants discovery that protozoal infections which are not responsive to therapy or prophylaxis with standard antiprotozoal agents, become sensitive to such therapy when administered in conjunction with a compound of formula 1. The effective amount of a formula I compound is from about 0.01 mg/kg of patient body weight per day to about 10 mg/kg of patient body weight per day and preferably from about 0.05 mg/kg day of patient body weight per day to about 5 mg/kg of patient body weight per day. A unit dosage form will conveniently contain, for example, from 0.50 to 100 mg of a formula I compound and one or more of such unit dosage forms can be administered 1 to 4 times daily.

The preferred route of administration is oral administration. For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or intraperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropyl-methylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylene-polypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

Pharmaceutical compositions of the antiprotozoal agents are widely available and can be used in the practice of this invention. For example, pharmaceutical compositions of antimalarial agents such as chloroquine and compositions of other antiprotozoal agents are also widely available and can be used in the practice of the method of this invention.

An illustrative composition for tablets is the following:

| | mg/tablet |
|---|---|
| (a) 2-(2-norbornylimino)azacyclo tridecane | 15 |
| (b) Lactose | 33 |
| (c) Corn starch | 11.25 |
| (d) Sucrose 3% starch | 12.75 |
| (e) Corn starch paste (10%) | 1.50 |
| (f) Zinc stearate | 1.50 |

The ability of the compounds of formula 1 to treat drug-resistant protozoal infections can be demonstrated by following the incorporation of [$^3$H]-hypoxanthine (Desjardins *et al*, (1979) Antimicrobial Agents Chemotherapy 16, 710-718) into drug-resistant Plasmodium *falciparum* using standard techniques. P. *falciparum* (clone D6, chloroquine-sensitive; strain FCR3, chloroquine-resistant; and clone W2, multidrug-resistant) was grown *in vitro* by the method of Trager and Jensen, (1976) *science* *193*, 673-675.

Figure 1 illustrates the synergism of 2-[(cis-2-phenyl-cyclopentyl)imino] azacyclotridecane hydrochloride and chloroquine against chloroquine-resistant *P. falciparum* (FCR3). Isobologram analysis was carried out according to the method of Martin, et al., (1987) science 235, 899-901 and Berenbaum,(1978) Journal of Infectious Diseases 137, 122-130. Drug synergy in this test is demonstrated when a test curve (solid line, test compound + chloroquine) falls to the left of the theoretical additivity line as is the case with the test compound + chloroquine curve. The X-axis is the $IC_{50}$ (concentration of drug which inhibits hypoxanthine incorporation by 50%) of chloroquine in the absence or presence of test compound while the Y-axis is the $IC_{50}$ for test compound in the absence or presence of chloroquine. The more highly synergistic a combination is, the closer to

the origin the points will fall. The combination of test compound and chloroquine is shown to be highly synergistic.

## Claims

**Claims for the following Contracting States : AT, BE, CH, DE, FR, GB, IT, LU, NL, SE**

1. A pharmaceutical composition comprising a lactamimide compound of the formula I

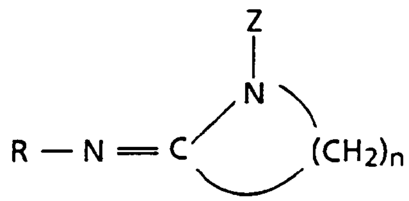

(I)

wherein

Z is hydrogen or lower alkyl of from 1 to 4 carbon atoms;

n is an integer of from 3 to 16;

R is

A) a straight or branched alkyl group of from 8 to 15 carbon atoms; or a lower alkoxyalkyl group wherein the alkyl moiety has from 8 to 15 carbon atoms and the alkoxy moiety has from 1 to 4 carbon atoms;

B) the group phenylalkyl wherein the alkyl moiety has from 1 to 6 carbon atoms and wherein the phenyl moiety is unsubstituted or substituted in which case the substituents may be attached at the ortho, meta or para-position of the phenyl ring and are chlorine, fluorine, bromine, lower alkyl having from 1 to 4 carbon atoms, lower alkoxy having from 1 to 4 carbon atoms, hydroxy or methylenedioxy;

C) the group

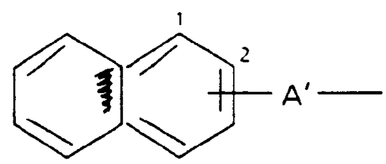

wherein A′ is a straight or branched alkylene group of from 1 to 6 carbon atoms or benzyl and is attached to either the 1- or 2-position of the naphthalene ring, which is either unsubstituted or is mono- or di-substituted with a chlorine, fluorine, bromine, trifluoromethyl, a straight or branched alkyl group of from 1 to 12 carbon atoms, an alkoxy group of from 1 to 3 carbon atoms or $NO_2$;

D) the group 1- or 2-adamantyl or l- or 2-norbornyl;

E) the group ortho, meta or para-biphenylyl;

F) the group 9-fluorenyl which is unsubstituted or substituted in which case the substituents may be attached to any one of the four available carbon atoms of each aromatic ring and are chlorine, bromine, fluorine, a lower alkyl group having from 1 to 4 carbon atoms, a lower alkoxy group having from 1 to 4 carbon atoms or $NO_2$;

G) dibenzocycloheptenyl;

H) the group

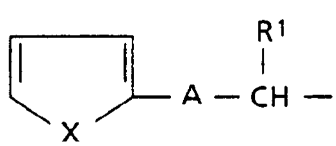

wherein X is oxygen or sulfur, A is a bond or an alkylene chain of from 1 to 3 carbon atoms, and $R^1$ is hydrogen, a straight or branched lower alkyl group having from 1 to 4 carbon atoms, a straight or

branched lower alkenyl group having from 3 to 6 carbon atoms, cycloalkyl of from 3 to 6 carbon atoms or phenyl;

I) the group

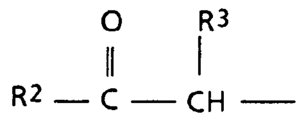

wherein $R^2$ is phenyl or a cycloalkyl group having from 3 to 6 carbon atoms, and $R^3$ is hydrogen or methyl;

J) the group

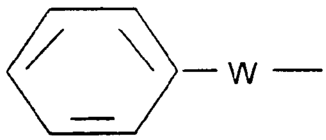

wherein W is a straight or branched alkylene chain having from 2 to 6 carbon atoms which is substituted with one phenyl group on any of the 6 carbon atoms with the proviso that the carbon atom adjacent to the exocyclic nitrogen atom must have at least one hydrogen attached to it;

K) the group

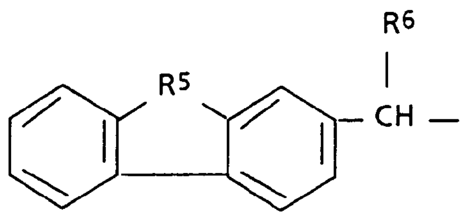

wherein $R^5$ is oxygen, sulfur, $-CH_2-$, $-CH_2CH_2-$ or-CH=CH-, and $R^6$ is hydrogen or a lower alkyl group of from 1 to 4 carbon atoms;

L) the group

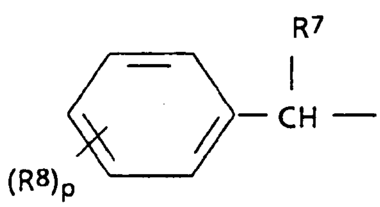

wherein $R^7$ is cycloalkyl of from 3 to 5 carbon atoms, $R^8$ is hydrogen, lower alkoxy of from 1 to 4 carbon atoms or lower alkyl of from 1 to 4 carbon atoms, and p is the integer 1 or 2;

M) the group

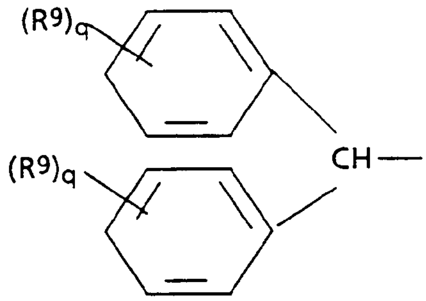

wherein $R^9$ is hydrogen, a straight or branched lower alkyl group of from 1 to 4 carbon atoms, chlorine, fluorine, bromine, $CF_3$, $SCF_3$, $OCF_3$, phenyl, phenoxy or a lower alkoxy group of from 1 to 4 carbon atoms and q is an integer of from 1 to 3;

N) the group

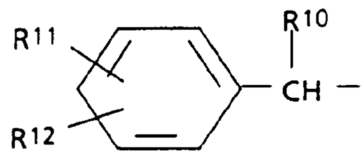

wherein $R^{10}$ is a lower alkyl group of from 1 to 4 carbon atoms, $R^{11}$ is hydrogen or lower alkyl of from 1 to 4 carbon atoms, $R^{12}$ is an alkyl group having from 8 to 14 carbon atoms, an alkoxy group having from 8 to 14 carbon atoms, a cycloalkyl group having from 5 to 14 carbon atoms, phenyl, phenoxy, phenylalkyl wherein the alkyl moiety has from 1 to 4 carbon atoms, phenylalkoxy wherein the alkoxy moiety has from 2 to 4 carbon atoms, 2,2-diphenylvinyl or fluoren-9-ylidene;

O) the group

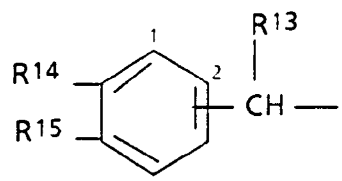

wherein the phenyl moiety is attached to the

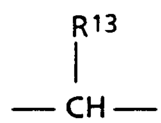

moiety through the 1- or 2-position of the phenyl ring, $R^{13}$ is a lower alkyl group of from 1 to 4 carbon atoms and $R^{14}$ and $R^{15}$ taken together are $(-CH_2-)_3$, $-CH_2CH_2C(CH_3)_2-$, $-(CH_2)_4-$ or $-C(CH_3)_2CH_2CH_2C(CH_3)_2-$;

P) the group

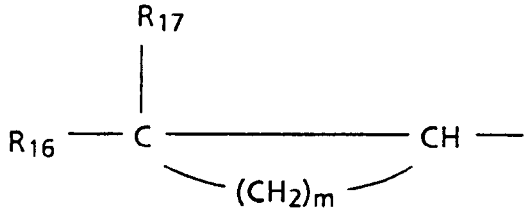

wherein $R_{16}$ is thienyl, cycloalkyl of from 5 to 7 carbon atoms, or is benzyl, phenyl or substituted

phenyl or benzyl, in which case the substitutents on the substituted phenyl are selected from fluorine, chlorine, bromine, iodine, lower alkyl of from 1 to 4 carbon atoms, and lower alkoxy of from 1 to 4 carbon atoms; Z is hydrogen or hydroxy; and m is an integer of from 3 to 6;

with the proviso that when R is the group

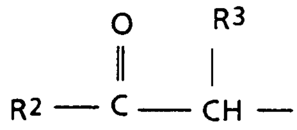

,

Z is hydrogen;

and pharmaceutically acceptable acid addition salts thereof, in conjunction with an antiprotozoal agent.

2. The composition of claim 1 wherein n is the integer 11.

3. The composition of claims 1 or 2 wherein R is 1- or 2-adamantyl or 1- or 2-norbornyl.

4. The composition of claim 3 wherein R is 2-norbornyl.

5. The composition of claim 1 wherein the compound is 2-(2-norbornylimino)azacyclotridecane or a pharmaceutically acceptable salt thereof.

6. The composition of claims 1 or 2 wherein R is

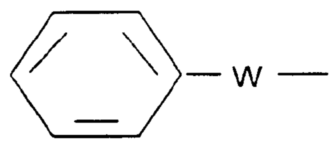

wherein

W is a straight or branched alkylene chain having from 2 to 6 carbon atoms which is substituted with one phenyl, 1-or 2-adamantyl, or l- or 2-norbornyl group on any one of the 6 carbon atoms with the proviso that the carbon atom adjacent to the exocyclic nitrogen atom must have at least 1 hydrogen attached thereto.

7. The composition of claims 1 or 2 wherein R is 2,2-diphenylpentyl.

8. The composition of claims 1 or 2 wherein R is α-(1-adamantyl)benzyl.

9. The composition of claims 1 or 2 wherein R is 3,3-phenylpropyl.

10. The composition of claims 1 or 2 wherein R is ortho, meta or parabiphenylyl.

11. The composition of claim 10 wherein R is ortho-biphenylyl.

12. The composition of claim 1 wherein the compound is 2-(o-biphenylylimino)azacyclotridecane or a pharmaceutically acceptable salt thereof.

13. The composition of claim 1 wherein the compound is 2-[(cis-2-phenylcyclopentyl)imino]azacyclotridecane or a pharmaceutically acceptable salt thereof.

14. The composition of any of claims 1 to 13 wherein the antiprotozoal agent is quinine, chloroquine, amodiaquine, primaquine, or mefloquine.

15. The composition of any of claims 1 to 14 for the separate sequential or concomitant use in the treatment or prevention of a drug-resistant protozoal infection with the antiprotozoal agent being present in amounts effective to treat or prevent the drug-resistant infection.

**16.** The composition of claim 15 wherein the drug-resistant protozoal infection is a drug-resistant malarial infection.

**17.** The composition of any of claims 1 to 16 together with a pharmaceutically acceptable carrier.

**Claims for the following Contracting State: GR**

**1.** Separate sequential or concomitant use of a lactamimide compound of the formula I

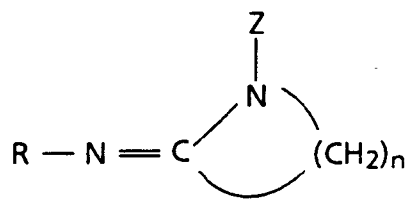

(I)

wherein

Z is hydrogen or lower alkyl of from 1 to 4 carbon atoms;
n is an integer of from 3 to 16;
R is

A) a straight or branched alkyl group of from 8 to 15 carbon atoms; or a lower alkoxyalkyl group wherein the alkyl moiety has from 8 to 15 carbon atoms and the alkoxy moiety has from 1 to 4 carbon atoms;
B) the group phenylalkyl wherein the alkyl moiety has from 1 to 6 carbon atoms and wherein the phenyl moiety is unsubstituted or substituted in which case the substituents may be attached at the ortho, meta or para-position of the phenyl ring and are chlorine, fluorine, bromine, lower alkyl having from 1 to 4 carbon atoms, lower alkoxy having from 1 to 4 carbon atoms, hydroxy or methylenedioxy;
C) the group

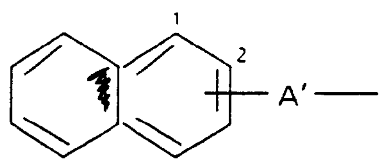

wherein A′ is a straight or branched alkylene group of from 1 to 6 carbon atoms or benzyl and is attached to either the 1- or 2-position of the naphthalene ring, which is either unsubstituted or is mono- or di-substituted with a chlorine, fluorine, bromine, trifluoromethyl, a straight or branched alkyl group of from 1 to 12 carbon atoms, an alkoxy group of from 1 to 3 carbon atoms or $NO_2$;
D) the group 1- or 2-adamantyl or 1- or 2-norbornyl;
E) the group ortho, meta or para-biphenylyl;
F) the group 9-fluorenyl which is unsubstituted or substituted in which case the substituents may be attached to any one of the four available carbon atoms of each aromatic ring and are chlorine, bromine, fluorine, a lower alkyl group having from 1 to 4 carbon atoms, a lower alkoxy group having from 1 to 4 carbon atoms or $NO_2$;
G) dibenzocycloheptenyl;
H) the group

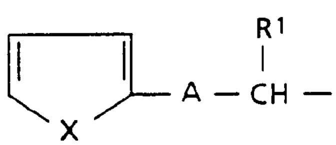

wherein X is oxygen or sulfur, A is a bond or an alkylene chain of from 1 to 3 carbon atoms, and $R^1$ is hydrogen, a straight or branched lower alkyl group having from l to 4 carbon atoms, a straight or branched lower alkenyl group having from 3 to 6 carbon atoms, cycloalkyl of from 3 to 6 carbon atoms

or phenyl;
I) the group

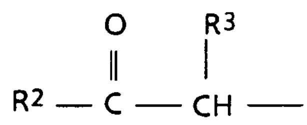

wherein $R^2$ is phenyl or a cycloalkyl group having from 3 to 6 carbon atoms, and $R^3$ is hydrogen or methyl;
J) the group

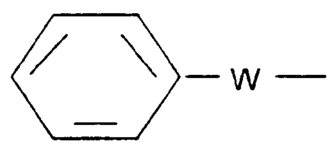

wherein W is a straight or branched alkylene chain having from 2 to 6 carbon atoms which is substituted with one phenyl group on any of the 6 carbon atoms with the proviso that the carbon atom adjacent to the exocyclic nitrogen atom must have at least one hydrogen attached to it;
K) the group

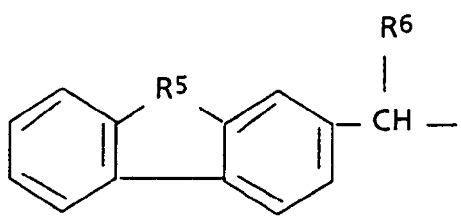

wherein $R^5$ is oxygen, sulfur, $-CH_2-$, $-CH_2CH_2-$ or $-CH=CH-$, and $R^6$ is hydrogen or a lower alkyl group of from 1 to 4 carbon atoms;
L) the group

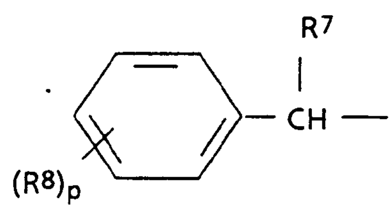

wherein $R^7$ is cycloalkyl of from 3 to 5 carbon atoms, $R^8$ is hydrogen, lower alkoxy of from 1 to 4 carbon atoms or lower alkyl of from 1 to 4 carbon atoms, and p is the integer 1 or 2;
M) the group

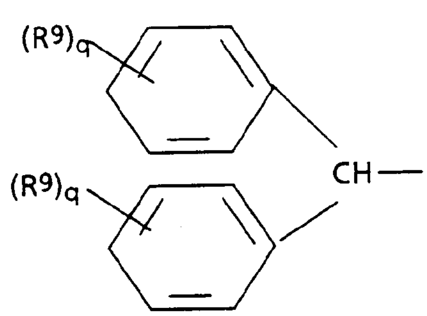

wherein $R^9$ is hydrogen, a straight or branched lower alkyl group of from 1 to 4 carbon atoms, chlorine, fluorine, bromine, $CF_3$, $SCF_3$, $OCF_3$, phenyl, phenoxy or a lower alkoxy group of from 1 to 4 carbon atoms and q is an integer of from 1 to 3;
N) the group

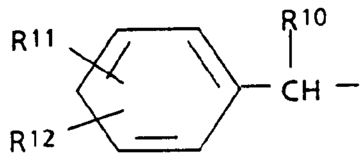

wherein $R^{10}$ is a lower alkyl group of from 1 to 4 carbon atoms, $R^{11}$ is hydrogen or lower alkyl of from 1 to 4 carbon atoms, $R^{12}$ is an alkyl group having from 8 to 14 carbon atoms, an alkoxy group having from 8 to 14 carbon atoms, a cycloalkyl group having from 5 to 14 carbon atoms, phenyl, phenoxy, phenylalkyl wherein the alkyl moiety has from 1 to 4 carbon atoms, phenylalkoxy wherein the alkoxy moiety has from 2 to 4 carbon atoms, 2,2-diphenylvinyl or fluoren-9-ylidene;
O) the group

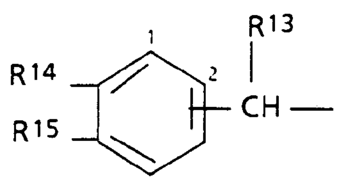

wherein the phenyl moiety is attached to the

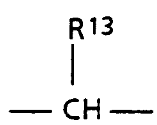

moiety through the 1- or 2-position of the phenyl ring, $R^{13}$ is a lower alkyl group of from 1 to 4 carbon atoms and $R^{14}$ and $R^{15}$ taken together are $(-CH_2-)_3$, $-CH_2CH_2C(CH_3)_2-$, $-(CH_2)_4-$ or $-C(CH_3)_2CH_2CH_2C(CH_3)_2-$;
P) the group

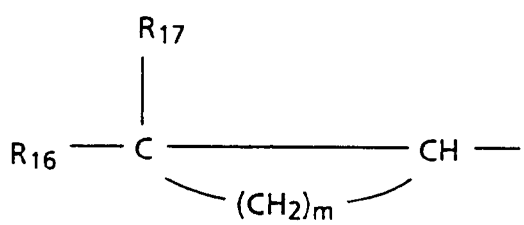

wherein $R_{16}$ is thienyl, cycloalkyl of from 5 to 7 carbon atoms, or is benzyl, phenyl or substituted phenyl or benzyl, in which case the substitutents on the substituted phenyl are selected from fluorine, chlorine, bromine, iodine, lower alkyl of from 1 to 4 carbon atoms, and lower alkoxy of from 1 to 4 carbon atoms; Z is hydrogen or hydroxy; and m is an integer of from 3 to 6;
with the proviso that when R is the group

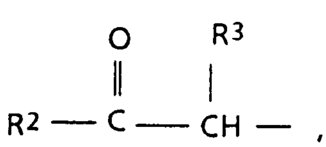

Z is hydrogen;
and pharmaceutically acceptable acid addition salts thereof,
in conjunction with an antiprotozoal agent for the preparation of a pharmaceutical composition.

2. Use according to claim 1 wherein n is the integer 11.

3. Use according to claims 1 or 2 wherein R is 1- or 2-adamantyl or 1- or 2-norbornyl.

4. Use according to claim 3 wherein R is 2-norbornyl.

5. Use according to claim 1 wherein the compound is 2-(2-norbornylimino)azacyclotridecane or a pharmaceutically acceptable salt thereof.

6. Use according to claims 1 or 2 wherein R is

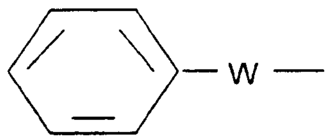

wherein
W is a straight or branched alkylene chain having from 2 to 6 carbon atoms which is substituted with one phenyl, 1-or 2-adamantyl, or 1- or 2-norbornyl group on any one of the 6 carbon atoms with the proviso that the carbon atom adjacent to the exocyclic nitrogen atom must have at least 1 hydrogen attached thereto.

7. Use according to claims 1 or 2 wherein R is 2,2-diphenylpentyl.

8. Use according to claims 1 or 2 wherein R is α-(1-adamantyl)benzyl.

9. Use according to claims 1 or 2 wherein R is 3,3-diphenylpropyl.

10. Use according to claims 1 or 2 wherein R is ortho, meta or parabiphenylyl.

11. Use according to claim 10 wherein R is ortho-biphenylyl.

12. Use according to claims 1 or 2 wherein the compound is 2-(o-biphenylylimino)azacyclotridecane or a pharmaceutically acceptable salt thereof.

13. Use according to claim 1 wherein the compound is 2-[(cis-2-phenylcyclopentyl)imino]azacyclotridecane or a pharmaceutically acceptable salt thereof.

14. Use according to any of claims 1 to 13 wherein the antiprotozoal agent is quinine, chloroquine, amodiaquine, primaquine, or mefloquine.

15. Use according to any of claims 1 to 14 wherein the pharmaceutical composition is a composition for the treatment or prevention of a drug-resistant infection and the antiprotozoal agent is added in amounts effective to treat the drug-resistant infection.

16. Use according to claim 15 wherein the drug-resistant protozoal infection is a drug-resistant malarial infection.

17. Use according to any of claims 1 to 16 wherein the composition contains additionally a pharmaceutically acceptable carrier.

**Claims for the following Contracting State: ES**

1. Method for the preparation of a composition comprising mixing a lactamimide compound of the formula I

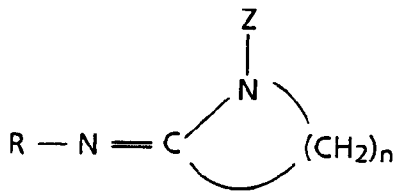

(I)

wherein

Z is hydrogen or lower alkyl of from 1 to 4 carbon atoms;
n is an integer of from 3 to 16;
R is

A) a straight or branched alkyl group of from 8 to 15 carbon atoms; or a lower alkoxyalkyl group wherein the alkyl moiety has from 8 to 15 carbon atoms and the alkoxy moiety has from 1 to 4 carbon atoms;
B) the group phenylalkyl wherein the alkyl moiety has from 1 to 6 carbon atoms and wherein the phenyl moiety is unsubstituted or substituted in which case the substituents may be attached at the ortho, meta or para-position of the phenyl ring and are chlorine, fluorine, bromine, lower alkyl having from 1 to 4 carbon atoms, lower alkoxy having from 1 to 4 carbon atoms, hydroxy or methylenedioxy;
C) the group

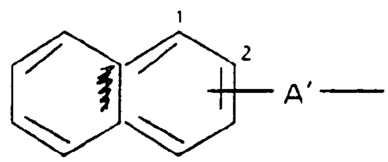

wherein A' is a straight or branched alkylene group of from 1 to 6 carbon atoms or benzyl and is attached to either the 1- or 2-position of the naphthalene ring, which is either unsubstituted or is mono- or di-substituted with a chlorine, fluorine, bromine, trifluoromethyl, a straight or branched alkyl group of from 1 to 12 carbon atoms, an alkoxy group of from 1 to 3 carbon atoms or $NO_2$;
D) the group 1- or 2-adamantyl or 1- or 2-norbornyl;
E) the group ortho, meta or para-biphenylyl;
F) the group 9-fluorenyl which is unsubstituted or substituted in which case the substituents may be attached to any one of the four available carbon atoms of each aromatic ring and are chlorine, bromine, fluorine, a lower alkyl group having from 1 to 4 carbon atoms, a lower alkoxy group having from 1 to 4 carbon atoms or $NO_2$;
G) dibenzocycloheptenyl;
H) the group

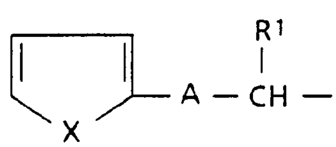

wherein X is oxygen or sulfur, A is a bond or an alkylene chain of from 1 to 3 carbon atoms, and $R^1$ is hydrogen, a straight or branched lower alkyl group having from 1 to 4 carbon atoms, a straight or branched lower alkenyl group having from 3 to 6 carbon atoms, cycloalkyl of from 3 to 6 carbon atoms or phenyl;
I) the group

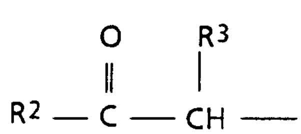

wherein $R^2$ is phenyl or a cycloalkyl group having from 3 to 6 carbon atoms, and $R^3$ is hydrogen or methyl;

J) the group

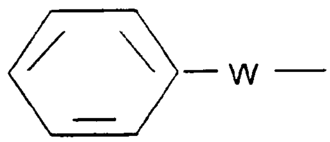

wherein W is a straight or branched alkylene chain having from 2 to 6 carbon atoms which is substituted with one phenyl group on any of the 6 carbon atoms with the proviso that the carbon atom adjacent to the exocyclic nitrogen atom must have at least one hydrogen attached to it;

K) the group

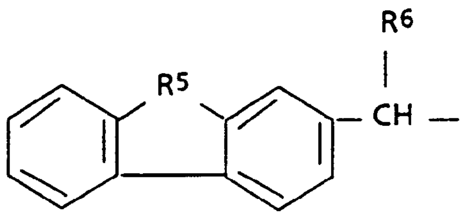

wherein $R^5$ is oxygen, sulfur, $-CH_2-$, $-CH_2CH_2-$ or-CH=CH-, and $R^6$ is hydrogen or a lower alkyl group of from 1 to 4 carbon atoms;

L) the group

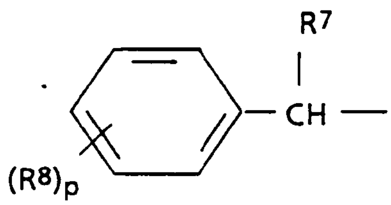

wherein $R^7$ is cycloalkyl of from 3 to 5 carbon atoms, $R^8$ is hydrogen, lower alkoxy of from 1 to 4 carbon atoms or lower alkyl of from 1 to 4 carbon atoms, and p is the integer 1 or 2;

M) the group

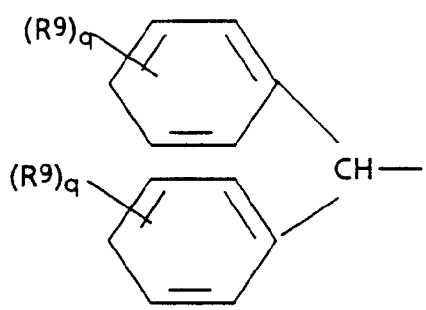

wherein $R^9$ is hydrogen, a straight or branched lower alkyl group of from 1 to 4 carbon atoms, chlorine, fluorine, bromine, $CF_3$, $SCF_3$, $OCF_3$, phenyl, phenoxy or a lower alkoxy group of from L to 4 carbon atoms and q is an integer of from 1 to 3;

N) the group

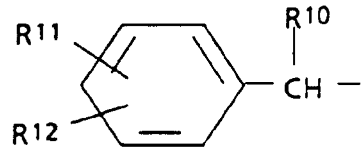

wherein $R^{10}$ is a lower alkyl group of from 1 to 4 carbon atoms, $R^{11}$ is hydrogen or lower alkyl of from 1 to 4 carbon atoms, $R^{12}$ is an alkyl group having from 8 to 14 carbon atoms, an alkoxy group having from 8 to 14 carbon atoms, a cycloalkyl group having from 5 to 14 carbon atoms, phenyl, phenoxy, phenylalkyl wherein the alkyl moiety has from 1 to 4 carbon atoms, phenylalkoxy wherein the alkoxy moiety has from 2 to 4 carbon atoms, 2,2-diphenylvinyl or fluoren-9-ylidene;

O) the group

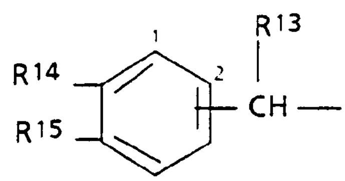

wherein the phenyl moiety is attached to the

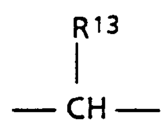

moiety through the 1- or 2-position of the phenyl ring, $R^{13}$ is a lower alkyl group of from 1 to 4 carbon atoms and $R^{14}$ and $R^{15}$ taken together are $(-CH_2-)_3$, $-CH_2CH_2C(CH_3)_2-$, $-(CH_2)_4-$ or $-C(CH_3)_2CH_2CH_2C(CH_3)_2-$;

P) the group

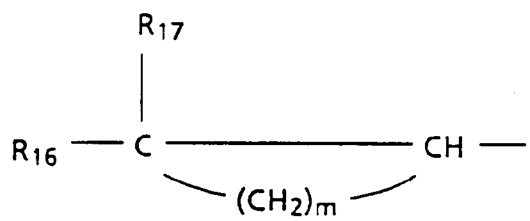

wherein $R_{16}$ is thienyl, cycloalkyl of from 5 to 7 carbon atoms, or is benzyl, phenyl or substituted phenyl or benzyl, in which case the substitutents on the substituted phenyl are selected from fluorine, chlorine, bromine, iodine, lower alkyl of from 1 to 4 carbon atoms, and lower alkoxy of from 1 to 4 carbon atoms; Z is hydrogen or hydroxy; and m is a integer of from 3 to 6;

with the proviso that when R is the group

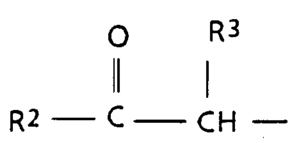 ,

Z is hydrogen;

and pharmaceutically acceptable acid addition salts thereof, with an antiprotozoal agent.

2. The method of claim 1 wherein n is the integer 11.

3. The method of claims 1 or 2 wherein R is 1- or 2-adamantyl or 1- or 2-norbornyl.

4. The method of claim 3 wherein R is 2-norbornyl.

5. The method of claim 1 wherein the compound is 2-(2-norbornylimino)azacyclotridecane or a pharmaceutically acceptable salt thereof.

6. The method of claims 1 or 2 wherein R is

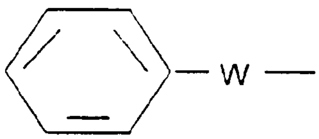

wherein

W is a straight or branched alkylene chain having from 2 to 6 carbon atoms which is substituted with one phenyl, 1-or 2-adamantyl, or 1- or 2-norbornyl group on any one of the 6 carbon atoms with the proviso that the carbon atom adjacent to the exocyclic nitrogen atom must have at least 1 hydrogen attached thereto.

7. The method of claims 1 or 2 wherein R is 2,2-diphenylpentyl.

8. The method of claims 1 or 2 wherein R is α-(1-adamantyl)benzyl.

9. The method of claims 1 or 2 wherein R is 3,3-diphenylpropyl.

10. The method of claims 1 or 2 wherein R is ortho, meta or parabiphenylyl.

11. The method of claim 10 wherein R is ortho-biphenyl.

12. The method of claims 1 or 2 wherein the compound is 2-(o-biphenylylimino)azacyclotridecane or a pharmaceutically acceptable salt thereof.

13. The method of claim 1 wherein the compound is 2-[(cis-2-phenylcyclopentyl)imino]azacyclotridecane or a pharmaceutically acceptable salt thereof.

14. The method of any of claims 1 to 13 wherein the antiprotozoal agent is quinine, chloroquine, amodiaquine, primaquine, or mefloquine.

15. The method of any of claims 1 to 14 wherein the composition contains additionally a pharmaceutically acceptable carrier.

**Patentansprüche**

**Patentansprüche für folgende Verstragsstaten : AT, BE, CH, DE, FR, GB, IT, LU, LI, NL, SE**

1. Arzneimittel, umfassend eine Lactamimidverbindung der Formel I

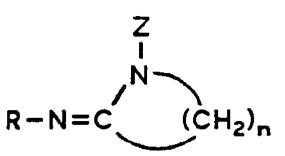

(I)

in der Z ein Wasserstoffatom oder einen Niederalkylrest mit 1 bis 4 Kohlenstoffatomen bedeutet;

n eine ganze Zahl von 3 bis 16 ist;

R

A) einen unverzweigten oder verzweigten Alkylrest mit 8 bis 15 Kohlenstoffatomen oder einen Niederalkoxyalkylrest, in dem die Alkyleinheit 8 bis 15 Kohlenstoffatome und die Alkoxyeinheit 1 bis 4 Kohlenstoffatome besitzt;

B) den Phenylolkylrest, in dem die Alkyleinheit 1 bis 6 Kohlenstoffatome besitzt, und in dem die- Phenyleinheit nichtsubstituiert oder substituiert ist, wobei die Substituenten in ortho-, meta- oder para-Stellung des Phenylringes gebunden sein können, und ein Chlor-, Fluor-, Bromatom, einen Niederalkylrest mit 1 bis 4 Kohlenstoffatomen, einen Niederalkoxyrest mit 1 bis 4 Kohlenstoffatomen, eine Hydroxy- ,oder Methylendioxygruppe bedeuten;

C) den Rest der Formel

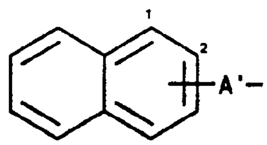

in der A′ ein unverzweigter oder verzweigter Alkylenrest mit 1 bis 6 Kohlenstoffatomen oder eine Benzylgruppe ist, und entweder in 1- oder 2-Stellung des Naphtholinringes gebunden ist, der entweder nichtsubstituiert oder mit einem Chlor-, Fluor-, Bromatom. einer Trifluormethylgruppe, einem unverzweigten oder verzweigten Alkylrest mit 1 bis 12 Kohlenstoffatomen, einem Alkoxyrest mit 1 bis 3 Kohlenstoffatomen oder einem Rest der Formel $NO_2$ mono- oder disubstituiert ist;

D) die 1- oder 2-Adamantyl- oder 1- oder 2- Norbornylgruppe;

E) die ortho-, meta- oder para-Biphenylylgruppe;

F) die 9-Fluorenylgruppe, die nichtsubstituiert oder substituiert ist, wobei die Substituenten an einen beliebigen der vier verfügbaren Kohlenstoffatome jedes aromatischen Ringes gebunden sein können, und ein Chlor-, Brom-, Fluoratom, einen Niederalkylrest mit 1 bis 4 Kohlenstoffatomen, einen Niederalkoxyrest mit 1 bis 4 Kohlenstoffatomen oder einen Rest der Formel $NO_2$ bedeuten;

G) eine Dibenzocycloheptenylgruppe;

H) den Rest der Formel

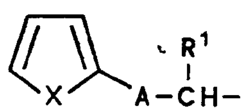

in der X ein Sauerstoff- oder Schwefelatom ist, A eine Bindung oder eine Alkylenkette mit 1 bis 3 Kohlenstoffatomen bedeutet, und $R^1$ ein Wasserstoffatom, einen unverzweigten oder verzweigten Niederalkylrest mit 1 bis 4 Kohlenstoffatomen, einen unverzweigten oder verzweigten Niederalkenylrest mit 3 bis 6 Kohlenstoffatomen, einen Cycloalkylrest mit 3 bis 6 Kohlenstoffatomen oder eine Phenylgruppe bedeutet;

I) den Rest der Formel

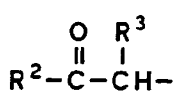

in der $R^2$ eine Phenylgruppe oder ein Cycloalkylrest mit 3 bis 6 Kohlenstoffatomen ist, und $R^3$ ein Wasserstoffatom oder eine Methylgruppe bedeutet;

J) den Rest der Formel

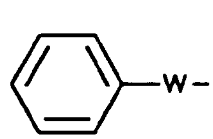

in der W eine unverzweigte oder verzweigte Alkylenkette mit 2 bis 6 Kohlenstoffatomen bedeutet, die mit einer Phenylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß;
K) den Rest der Formel

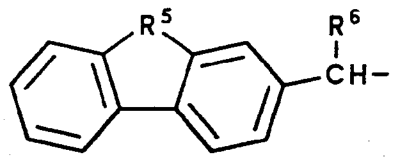

in der $R^5$ ein Sauerstoff-, Schwefelatom, einen Rest der Formel $-CH_2-$, $-CH_2CH_2-$ oder $-CH=CH-$ bedeutet, und $R^6$ ein Wasserstoffatom oder ein Niederalkylrest mit 1 bis 4 Kohlenstoffatomen ist;
L) den Rest der Formel

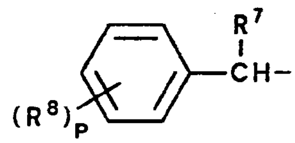

in der $R^7$ ein Cycloalkylrest mit 3 bis 5 Kohlenstoffatomen ist, Rs ein Wasserstoffatom, einen Niederalkoxyrest mit 1 bis 4 Kohlenstoffatomen oder einen Niederalkylrest mit 1 bis 4 Kohlenstoffatomen bedeutet, und p die ganze Zahl 1 oder 2 ist;
M) den Rest der Formel

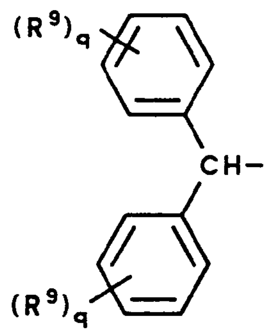

in der $R^9$ ein Wasserstoffatom, einen unverzweigten oder verzweigten Niederalkylrest mit 1 bis 4 Kohlenstoffatomen, ein Chlor-, Fluor-, Bromatom, einen Rest der Formel $CF_3$, $SCF_3$, $OCF_3$, eine Phenyl-, Phenoxygruppe oder einen Niederalkoxyrest mit 1 bis 4 Kohlenstoffatomen bedeutet, und q eine ganze Zahl von 1 bis 3 ist;
N) den Rest der Formel

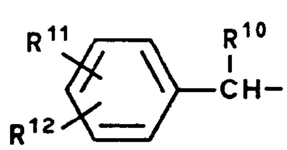

in der $R^{10}$ ein Niederalkylrest mit 1 bis 4 Kohlenstoffatomen ist, $R^{11}$ ein Wasserstoffatom oder einen Niederalkylrest mit 1 bis 4 Kohlenstoffatomen bedeutet, $R^{12}$ ein Alkylrest mit 8 bis 14 Kohlenstoffatomen, ein Alkoxyrest mit 8 bis 14 Kohlenstoffatomen, ein Cycloalkylrest mit 5 bis 14 Kohlenstoffatomen, eine Phenyl-, Phenoxygruppe, ein Phenylolkylrest, in dem die Alkyleinheit 1 bis 4 Kohlenstoffatome besitzt, ein Phenylalkoxyrest, in dem die Alkoxyeinheit 2 bis 4 Kohlenstoffatome besitzt, eine 2,2-Diphenylvinyl- oder eine Fluoren-9-ylidengruppe ist;
O) den Rest der Formel

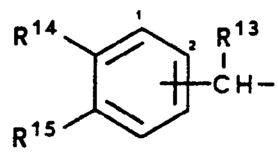

in der die Phenyleinheit über die 1- oder 2-Stellung des Phenylringes an die Einheit der Formel

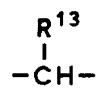

gebunden ist, $R^{13}$ ein Niederalkylrest mit 1 bis 4 Kohlenstoffatomen ist, und $R^{14}$ und $R^{15}$ zusammengenommen ein Rest der Formel $(-CH_2-)_3$, $-CH_2CH_2C(CH_3)_2-$, $-(CH_2)_4-$ oder $-C(CH_3)_2CH_2CH_2C(CH_3)_2-$ sind;
P) den Rest der Formel

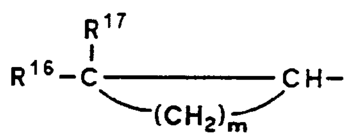

in der $R^{16}$ eine Thienylgruppe, einen Cycloalkylrest mit 5 bis 7 Kohlenstoffatomen oder eine Benzyl-, Phenyl- oder eine substituierte Phenyl- oder Benzylgruppe bedeutet, wobei die Substituenten an der substituierten Phenylgruppe aus einem Fluor-, Chlor-, Brom-, Iodatom, einem Niederalkylrest mit 1 bis 4 Kohlenstoffatomen und einem Niederalkoxyrest mit 1 bis 4 Kohlenstoffatomen ausgewählt sind, Z ein Wasserstoffatom oder eine Hydroxylgruppe ist, und m eine ganze Zahl von 3 bis 6 bedeutet; mit der Maßgabe, daß Z ein Wasserstoffatom ist, falls R der Rest der Formel

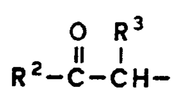

ist; bedeutet,
und pharmazeutisch verträgliche Säureadditionssalze davon zusammen mit einem Antiprotozoenwirkstoff.

**2.** Mittel noch Anspruch 1, wobei n die ganze Zahl 11 ist.

**3.** Mittel noch den Ansprüchen 1 oder 2, wobei R eine 1- oder 2-Adamantyl- oder eine 1- oder 2-Norbornylgruppe bedeutet.

**4.** Mittel noch Anspruch 3, wobei R eine 2-Norbornylgruppe bedeutet.

**5.** Mittel nach Anspruch 1, wobei die Verbindung 2-(2-Norbornylimino)azacyclotridecan oder ein pharmazeutisch verträgliches Salz davon ist.

**6.** Mittel nach den Ansprüchen 1 oder 2, wobei R einen Rest der Formel

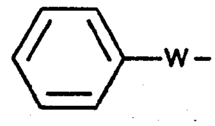

bedeutet, in der
W eine unverzweigte oder verzweigte Alkylenkette mit 2 bis 6 Kohlenstoffatomen ist, die mit einer Phenyl-, einer 1- oder 2-Adamantyl- oder einer 1- oder 2-Norbornylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß.

7. Mittel noch den Ansprüchen 1 oder 2, wobei R eine 2,2-Diphenylpentylgruppe bedeutet.

8. Mittel noch den Ansprüchen 1 oder 2, wobei R eine α-(1-Adamantyl)benzylgruppe bedeutet.

9. Mittel noch den Ansprüchen 1 oder 2, wobei R eine 3,3-Diphenylpropylgruppe bedeutet.

10. Mittel nach den Ansprüchen 1 oder 2, wobei R eine ortho-, meta- oder para-Biphenylylgruppe bedeutet.

11. Mittel nach Anspruch 10, wobei R eine ortho-Biphenylylgruppe bedeutet.

12. Mittel noch Anspruch 1, wobei die Verbindung 2-(o-Biphenylylimino)azacyclotridecan oder ein pharmazeutisch verträgliches Salz davon ist.

13. Mittel noch Anspruch 1, wobei die Verbindung 2-[(cis-2-Phenylcyclopentyl)iminolazacyclotridecan oder ein pharmazeutisch verträgliches Salz davon ist.

14. Mittel noch einem der Ansprüche 1 bis 13, wobei der Antiprotozoenwirkstoff Chinin, Chloroquin, Amodiaquin, Primaquin oder Mefloquin ist.

15. Mittel noch einem der Ansprüche 1 bis 14 zur einzelnen, aufeinanderfolgenden oder gleichzeitigen Verwendung bei der Behandlung oder Vorbeugung einer Arzneistoff-resistenten Protozoeninfektion, wobei der Antiprotozoenwirkstoff in zur Behandlung oder Vorbeugung der Arzneistoff-resistenten infektion wirksamen Mengen vorliegt.

16. Mittel noch Anspruch 15, wobei die Arzneistoff-resistente Protozoeninfektion eine Arzneistoff-resistente Malariainfektion ist.

17. Mittel nach einem der Ansprüche 1 bis 16 zusammen mit einem pharmazeutisch verträglichen Träger.

**Patentansprüche für folgenden Vertragsstaat : GR**

1. Einzelne, aufeinanderfolgende oder gleichzeitige Verwendung einer Lactamimidverbindung der Formel I

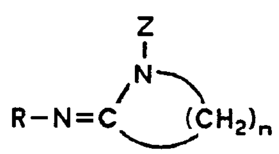

(I)

in der Z ein Wasserstoffatom oder einen Niederalkylrest mit 1 bis 4 Kohlenstoffatomen bedeutet;
n eine ganze Zahl von 3 bis 16 ist;
R
A) einen unverzweigten oder verzweigten Alkylrest mit 8 bis 15 Kohlenstoffatomen oder einen Niederalkoxyalkylrest, in dem die Alkyleinheit 8 bis 15 Kohlenstoffatome und die Alkoxyeinheit 1 bis 4 Kohlenstoffatome besitzt;
B) den Phenylolkylrest, in dem die Alkyleinheit 1 bis 6 Kohlenstoffatome besitzt, und in dem die Phenyleinheit nichtsubstituiert oder substituiert ist, wobei die Substituenten in ortho-, meta- oder para-Stellung des Phenylringes gebunden sein können, und ein Chlor-, Fluor-, Bromatom. einen Niederalkylrest mit 1 bis 4 Kohlenstoffatomen, einen Niederalkoxyrest mit 1 bis 4 Kohlenstoffatomen, eine Hydroxy- oder Methylendioxygruppe bedeuten;
C) den Rest der Formel

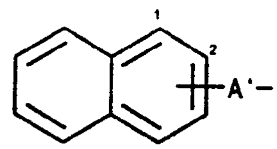

in der A′ ein unverzweigter oder verzweigter Alkylenrest mit 1 bis 6 Kohlenstoffatomen oder eine Benzylgruppe ist, und entweder in 1- oder 2-Stellung des Naphthalinringes gebunden ist, der entweder nichtsubstituiert oder mit einem Chlor-, Fluor-, Bromatom, einer Trifluormethylgruppe, einem unverzweigten oder verzweigten Alkylrest mit 1 bis 12 Kohlenstoffatomen, einem Alkoxyrest mit 1 bis 3 Kohlenstoffatomen oder einem Rest der Formel $NO_2$ mono- oder disubstituiert ist;
D) die 1- oder 2-Adamantyl- oder 1- oder 2- Norbornylgruppe;
E) die ortho-, meta- oder para-Biphenylylgruppe;
F) die 9-Fluorenylgruppe, die nichtsubstituiert oder substituiert ist, wobei die Substituenten an einen beliebigen der vier verfügbaren Kohlenstoffatome jedes aromatischen Ringes gebunden sein können, und ein Chlor-, Brom-, Fluoratom, einen Niederalkylrest mit 1 bis 4 Kohlenstoffatomen, einen Niederalkoxyrest mit 1 bis 4 Kohlenstoffatomen oder einen Rest der Formel $NO_2$ bedeuten;
G) eine Dibenzocycloheptenylgruppe;
H) den Rest der Formel

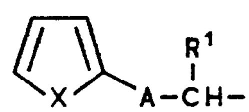

in der X ein Sauerstoff- oder Schwefelatom ist, A eine Bindung oder eine Alkylenkette mit 1 bis 3 Kohlenstoffatomen bedeutet, und Rl ein Wasserstoffatom, einen unverzweigten oder verzweigten Niederalkylrest mit 1 bis 4 Kohlenstoffatomen, einen unverzweigten oder verzweigten Niederalkenylrest mit 3 bis 6 Kohlenstoffatomen, einen Cycloalkylrest mit 3 bis 6 Kohlenstoffatomen oder eine Phenylgruppe bedeutet;
I) den Rest der Formel

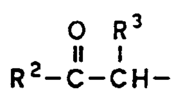

in der $R^2$ eine Phenylgruppe oder ein Cycloalkylrest mit 3 bis 6 Kohlenstoffatomen ist, und $R^3$ ein Wasserstoffatom oder eine Methylgruppe bedeutet;
J) den Rest der Formel

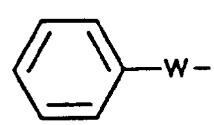

in der W eine unverzweigte oder verzweigte Alkylenkette mit 2 bis 6 Kohlenstoffatomen bedeutet, die mit einer Phenylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß;

K) den Rest der Formel

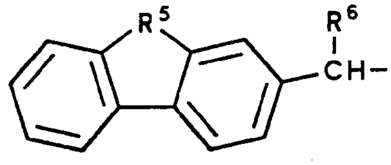

in der Rs ein Sauerstoff-, Schwefelatom, einen Rest der Formel $-CH_2-$, $-CH_2CH_2-$ oder $-CH=CH-$ bedeutet, und $R^6$ ein Wasserstoffatom oder -ein Niederalkylrest mit 1 bis 4 Kohlenstoffatomen ist;
L) den Rest der Formel

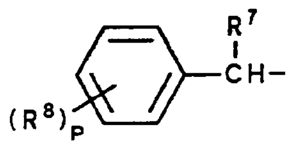

in der $R^7$ ein Cycloalkylrest mit 3 bis 5 Kohlenstoffatomen ist, $R^8$ ein Wasserstoffatom, einen Niederalkoxyrest mit 1 bis 4 Kohlenstoffatomen oder einen Niederalkylrest mit 1 bis 4 Kohlenstoffatomen bedeutet, und p die ganze Zahl 1 oder 2 ist;
M) den Rest der Formel

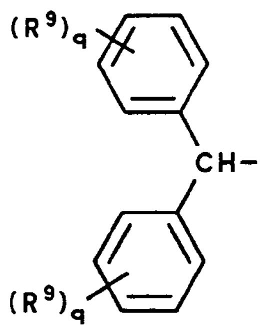

in der $R^9$ ein Wasserstoffatom, einen unverzweigten oder verzweigten Niederalkylrest mit 1 bis 4 Kohlenstoffatomen, ein Chlor-, Fluor-, Bromatom, einen Rest der Formel $CF_3$, $SCF_3$, $OCF_3$, eine Phenyl-, Phenoxygruppe oder einen Niederalkoxyrest mit 1 bis 4 Kohlenstoffatomen bedeutet, und q eine ganze Zahl von 1 bis 3 ist;
N) den Rest der Formel

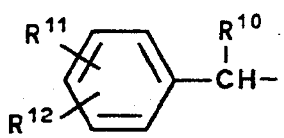

in der $R^{10}$ ein Niederalkylrest mit 1 bis 4 Kohlenstoffatomen ist, $R^{11}$ ein Wasserstoffatom oder einen Niederalkylrest mit 1 bis 4 Kohlenstoffatomen bedeutet, $R^{12}$ ein Alkylrest mit 8 bis 14 Kohlenstoffatomen, ein Alkoxyrest mit 8 bis 14 Kohlenstoffatomen, ein Cycloalkylrest mit 5 bis 14 Kohlenstoffatomen, eine Phenyl-, Phenoxygruppe, ein Phenylalkylrest, in dem die Alkyleinheit 1 bis 4 Kohlenstoffatome besitzt, ein Phenylalkoxyrest, in dem die Alkoxyeinheit 2 bis 4 Kohlenstoffatome besitzt, eine 2,2-Diphenylvinyl- oder eine Fluoren-9-ylidengruppe ist;
O) den Rest der Formel

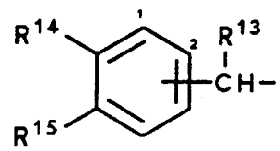

in der die Phenyleinheit über die 1- oder 2-Stellung des Phenylringes an die Einheit der Formel

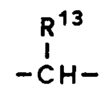

gebunden ist, $R^{13}$ ein Niederalkylrest mit 1 bis 4 Kohlenstoffatomen ist, und $R^{14}$ und $R^{15}$ zusammengenommen ein Rest der Formel $(-CH_2-)_3$, $-CH_2CH_2C(CH_3)_2-$, $-(CH_2)_4-$ oder $-C(CH_2)_2CH_2CH_2C(CH_3)_2-$ sind;
P) den Rest der Formel

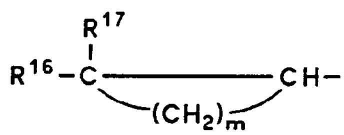

in der $R^{16}$ eine Thienylgruppe, einen Cycloalkylrest mit 5 bis 7 Kohlenstoffatomen oder eine Benzyl-, Phenyl- oder eine substituierte Phenyl- oder Benzylgruppe bedeutet, wobei die Substituenten an der substituierten Phenylgruppe aus einem Fluor-, Chlor -, Brom-, Iodatom, einem Niederalkylrest mit 1 bis 4 Kohlenstoffatomen und einem Niederalkoxyrest mit 1 bis 4 Kohlenstoffatomen ausgewählt sind, Z ein Wasserstoffatom oder eine Hydroxylgruppe ist, und m eine ganze Zahl von 3 bis 6 bedeutet; mit der Maßgabe, daß Z ein Wasserstoffatom ist, falls R der Rest der Formel

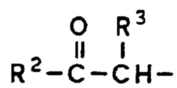

ist; bedeutet,
und pharmazeutisch verträgliche Säureudditionssolze davon zusammen mit einem Antiprotozoenwirkstoff zur Herstellung eines Arzneimittels.

**2.** Verwendung gemäß Anspruch 1, wobei n die ganze Zahl 11 ist.

**3.** Verwendung gemäß den Ansprüchen 1 oder 2, wobei R eine 1- oder 2-Adamantyl- oder eine 1- oder 2-Norbornylgruppe bedeutet.

**4.** Verwendung gemäß Anspruch 3, wobei R eine 2-Norbornylgruppe bedeutet.

**5.** Verwendung gemäß Anspruch 1, wobei die Verbindung 2-(2-Norbornylimino)azacyclotridecan oder ein pharmazeutisch verträgliches Salz davon ist.

**6.** Verwendung gemäß den Ansprüchen 1 oder 2, wobei R einen Rest der Formel

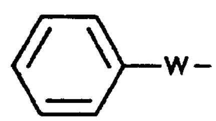

bedeutet, in der
W eine unverzweigte oder verzweigte Alkylenkette mit 2 bis 6 Kohlenstoffatomen ist, die mit einer Phenyl-, einer 1- oder 2-Adamantyl- oder einer 1- oder 2-Norbornylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß.

7. Verwendung gemäß den Ansprüchen 1 oder 2, wobei R eine 2,2-Diphenylpentylgruppe bedeutet.

8. Verwendung gemäß den Ansprüchen 1 oder 2, wobei R eine $\alpha$-(1-Adamantyl)benzylgruppe bedeutet.

9. Verwendung gemäß den Ansprüchen 1 oder 2, wobei R eine 3,3-Diphenylpropylgruppe bedeutet.

10. Verwendung gemäß den Ansprüchen 1 oder 2, wobei R eine ortho-, meta- oder para-Biphenylylgruppe bedeutet.

11. Verwendung gemäß Anspruch 10, wobei R eine ortho-Biphenylylgruppe bedeutet.

12. Verwendung gemäß den Ansprüchen 1 oder 2, wobei die Verbindung 2-(o-Biphenylylimino)azacyclotridecan oder ein pharmozeutisch verträgliches Salz davon ist.

13. Verwendung gemäß Anspruch 1, wobei die Verbindung 2-[(cis-2-Phenylcyclopentyl)imino]azacyclotridecan oder ein pharmazeutisch verträgliches Salz davon ist.

14. Verwendung gemäß einem der Ansprüche 1 bis 13, wobei der Antiprotozoenwirkstoff Chinin, Chloroquin, Amodiaquin, Primaquin oder Mefloquin ist.

15. Verwendung gemäß einem der Ansprüche 1 bis 14, wobei das Arzneimittel eine Zusammensetzung zur Behandlung oder Vorbeugung einer Arzneistoff-resistenten Infektion ist, und der Antiprotozoenwirkstoff in- zur Behandlung der Arzneistoff-resistenten Infektion wirksamen Mengen zugegeben wird.

16. Verwendung gemäß Anspruch 15, wobei die Arzneistoffresistente Protozoeninfektion eine Arzneistoff-resistente Malariainfektion ist.

17. Verwendung gemäß einem der Ansprüche 1 bis 16, wobei die Zusammensetzung ferner einen pharmazeutisch verträglichen Träger enthält.

**Patentansprüche für folgenden Vertragsstaat : ES**

1. Verfahren zur Herstellung einer Zusammensetzung, umfassend das Mischen einer Lactamimidverbindung der Formel I

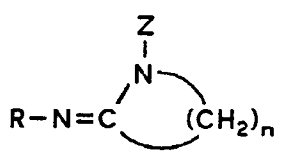

(I)

in der Z ein Wasserstoffatom oder einen Niederalkylrest mit 1 bis 4 Kohlenstoffatomen bedeutet;
n eine ganze Zahl von 3 bis 16 ist;
R
A) einen unverzweigten oder verzweigten Alkylrest mit 8 bis 15 Kohlenstoffatomen oder einen Niederalkoxyalkylrest, in dem die Alkyleinheit 8 bis 15 Kohlenstoffatome und die Alkoxyeinheit 1 bis 4 Kohlenstoffatome besitzt;
B) den Phenylalkylrest, in dem die Alkyleinheit 1 bis 6 Kohlenstoffatome besitzt, und in dem die Phenyleinheit nichtsubstituiert oder substituiert ist, wobei die Substituenten in ortho-, meta- oder para-Stellung des- Phenylringes gebunden sein können, und ein Chlor-, Fluor-, Bromatom, einen Niederalkylrest mit 1 bis 4 Kohlenstoffatomen, einen Niederalkoxyrest mit 1 bis 4 Kohlenttoffatomen, eine Hydroxy- oder Methylendioxygruppe bedeuten;
C) den Rest der Formel

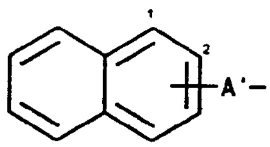

in der A′ ein unverzweigter oder verzweigter Alkylenrest mit 1 bis 6 Kohlenstoffatomen oder eine Benzylgruppe ist, und entweder in 1- oder 2-Stellung des Naphtholinringes gebunden ist, der entweder nichtsubstituiert oder mit einem Chlor-, Fluor-, Bromatom, einer Trifluormethylgruppe, einem unverzweigten oder verzweigten Alkylrest mit 1 bis 12 Kohlenstoffatomen, einem Alkoxyrest mit 1 bis 3 Kohlenstoffatomen oder einem Rest der Formel $NO_2$ mono- oder disubstituiert ist;
D) die 1- oder 2-Adamantyl- oder 1- oder 2- Norbornylgruppe;
E) die ortho-, meta- oder para-Biphenylylgruppe;
F) die 9-Fluorenylgruppe, die nichtsubstituiert oder substituiert ist, wobei die Substituenten an einen beliebigen der vier verfügbaren Kohlenstoffatome jedes aromatischen Ringes gebunden sein können, und ein Chlor-, Brom-, Fluoratom, einen Niederalkylrest mit 1 bis 4 Kohlenstoffatomen, einen Niederalkoxyrest mit 1 bis 4 Kohlenstoffatomen oder einen Rest der Formel $NO_2$ bedeuten;
G) eine Dibenzocycloheptenylgruppe;
H) den Rest der Formel

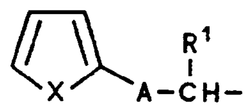

in der X ein Sauerstoff- oder Schwefelatem ist, A eine Bindung oder eine Alkylenkette mit 1 bis 3 Kohlenstoffatomen bedeutet, und $R^1$ ein Wasserstoffatom, einen unverzweigten oder verzweigten Niederalkylrest mit 1 bis 4 Kohlenstoffatomen, einen unverzweigten oder verzweigten Niederalkenylrest mit 3 bis 6 Kohlenstoffatomen, einen Cycloalkylrest mit 3 bis 6 Kohlenstoffatomen oder eine Phenylgruppe bedeutet;
I) den Rest der Formel

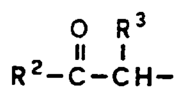

in der $R^2$ eine Phenylgruppe oder ein Cycloalkylrest mit 3 bis 6 Kohlenstoffatomen ist, und $R^3$ ein Wasserstoffatom oder eine Methylgruppe bedeutet;
J) den Rest der Formel

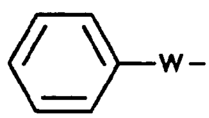

in der W eine unverzweigte oder verzweigte Alkylenkette mit 2 bis 6 Kohlenstoffatomen bedeutet, die mit einer Phenylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß;
K) den Rest der Formel

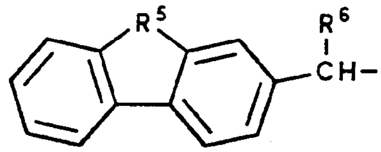

in der $R^5$ ein Sauerstoff-, Schwefelatom, einen Rest der Formel $-CH_2-$, $-CH_2CH_2-$ oder $-CH=CH-$ bedeutet, und $R^6$ ein Wasserstoffatom oder ein Niederalkylrest mit 1 bis 4 Kohlenstoffatomen ist;
L) den Rest der Formel

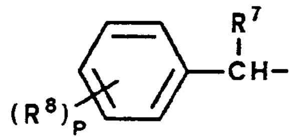

in der $R^7$ ein Cycloalkylrest mit 3 bis 5 Kohlenstoffatomen ist, $R^8$ ein Wasserstoffatom, einen Niederalkoxyrest mit 1 bis 4 Kohlenstoffatomen oder einen Niederalkylrest mit 1 bis 4 Kohlenstoffatomen bedeutet, und p die ganze Zahl 1 oder 2 ist;
M) den Rest der Formel

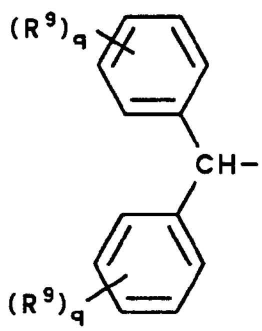

in der $R^9$ ein Wasserstoffatom, einen unverzweigten oder verzweigten Niederalkylrest mit 1 bis 4 Kohlenstoffatomen, ein Chlor-, Fluor-, Bromatom, einen Rest der Formel $CF_3$, $SCF_3$, $OCF_3$, eine Phenyl-, Phenoxygruppe oder einen Niederalkoxyrest mit 1 bis 4 Kohlenstoffatomen bedeutet, und q eine ganze Zahl von 1 bis 3 ist;
N) den Rest der Formel

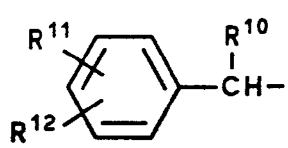

in der $R^{10}$ ein Niederalkylrest mit 1 bis 4 Kohlenstoffatomen ist, $R^{11}$ ein Wasserstoffatom oder einen Niederalkylrest mit 1 bis 4 Kohlenstoffatomen bedeutet, $R^{12}$ ein Alkylrest mit 8 bis 14 Kohlenstoffatomen, ein Alkoxyrest mit 8 bis 14 Kohlenstoffatomen, ein Cycloalkylrest mit 5 bis 14 Kohlenstoffatomen, eine Phenyl-, Phenoxygruppe, ein Phenylalkylrest, in dem die Alkyleinheit 1 bis 4 Kohlenstoffatome besitzt, ein Phenylalkoxyrest, in dem die Alkoxyeinheit 2 bis 4 Kohlenstoffatome besitzt, eine 2,2-Diphenylvinyl- oder eine Fluoren-9-ylidengruppe ist;
O) den Rest der Formel

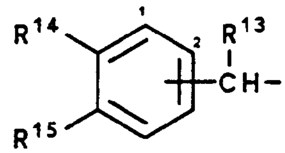

in der die Phenyleinheit über die 1- oder 2-Stellung des Phenylringes an die Einheit der Formel

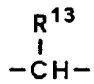

gebunden ist, $R^{13}$ ein Niederalkylrest mit 1 bis 4 Kohlenstoffatomen ist, und $R^{14}$ und $R^{15}$ zusammengenommen ein Rest der Formel $(-CH_2-)_3$, $-CH_2CH_2C(CH_3)_2-$. $-(CH_2)_4-$ oder $-C(CH_3)_2CH_2CH_2C(CH_3)_2-$ sind;
P) den Rest der Formel

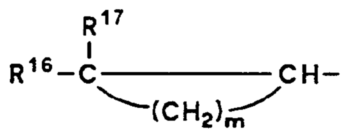

in der $R^{16}$ eine Thienylgruppe, einen Cycloalkylrest mit 5 bis 7 Kohlenstoffatomen oder eine Benzyl-, Phenyl- oder eine substituierte Phenyl- oder Benzylgruppe bedeutet, wobei die Substituenten an der substituierten -Phenylgruppe aus einem Fluor-, Chlor-, Brom- Iodatom, einem Niederalkylrest mit 1 bis 4 Kohlenstoffatomen und einem Niederalkoxyrest mit 1 bis 4 Kohlenstoffatomen ausgewählt sind, Z ein Wasserstoffatom oder eine Hydroxylgruppe ist, und m eine ganze Zahl von 3 bis 6 bedeutet; mit der Maßgabe, daß Z ein Wasserstoffatom ist, falls R der Rest der Formel

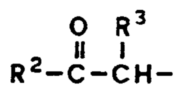

ist; bedeutet,
und pharmazeutisch verträgliche Säureadditionssalze davon mit einem Antiprotozoenwirkstoff.

2. Verfahren nach Anspruch 1, wobei n die ganze Zahl 11 ist.

3. Verfahren nach den Ansprüchen 1 oder 2, wobei R eine 1- oder 2-Adamantyl- oder eine 1- oder 2-Norbornylgruppe bedeutet.

4. Verfahren noch Anspruch 3, wobei R eine 2-Norbornylgruppe bedeutet.

5. Verfahren noch Anspruch 1, wobei die Verbindung 2-(2-Norbornylimino)azacyclotridecan oder ein pharmazeutisch verträgliches Salz davon ist.

6. Verfahren noch den Ansprüchen 1 oder 2, wobei R einen Rest der Formel

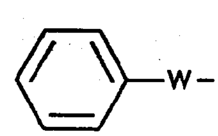

bedeutet, in der
W eine unverzweigte oder verzweigte Alkylenkette mit 2 bis 6 Kohlenstoffatomen ist, die mit einer Phenyl-, einer 1- oder 2-Adamantyl- oder einer 1- oder 2-Norbornylgruppe an einem der 6 Kohlenstoffatome substituiert ist, mit der Maßgabe, daß an das Kohlenstoffatom, das dem exocyclischen Stickstoffatom benachbart ist, mindestens ein Wasserstoffatom gebunden sein muß.

7. Verfahren noch den Ansprüchen 1 oder 2, wobei R eine 2,2-Diphenylpentylgruppe bedeutet.

8. Verfahren nach den Ansprüchen 1 oder 2, wobei R eine α-(1-Adamantyl)benzylgruppe bedeutet.

9. Verfahren noch den Ansprüchen 1 oder 2, wobei R eine 3,3-Diphenylpropylgruppe bedeutet.

10. Verfahren nach den Ansprüchen 1 oder 2, wobei R eine ortho-, meta- oder para-Biphenylylgruppe bedeutet.

11. Verfahren nach Anspruch 10, wobei R eine ortho-Biphenylgruppe bedeutet.

12. Verfahren nach den Ansprüchen 1 oder 2, wobei die Verbindung 2-(o-Biphenylylimino)azacyclotridecan oder ein pharmazeutisch verträgliches Salz davon ist.

13. Verfahren noch Anspruch 1, wobei die Verbindung 2-[(cis-2-Phenylcyclopentyl)iminolazacyclotridecan oder ein pharmazeutisch verträgliches Salz davon ist.

14. Verfahren nach einem der Ansprüche 1 bis 13, wobei der Antiprotozoenwirkstoff Chinin, Chloroquin, Amodiaquin, Primaquin oder Mefloquin ist.

15. Verfahren noch einem der Ansprüche 1 bis 14, wobei die Zusammensetzung ferner einen pharmazeutisch verträglichen Träger enthält.

## Revendications

### Revendications pour les Etats Contractants suivants : AT, BE, CH, DE, FR, GB, IT, LI, LU, NL, SE

1. Composition pharmaceutique comprenant un lactamimide de formule I

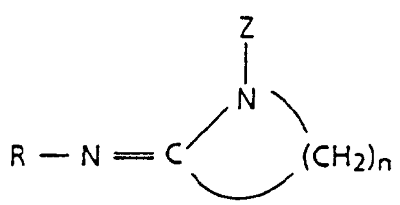

(I)

dans laquelle
Z représente un atome d'hydrogène ou un groupe alkyle inférieur ayant 1 à 4 atomes de carbone ;
n est un nombre entier valant 3 à 16 ;
R représente :

A) un groupe alkyle, linéaire ou ramifié, ayant 8 à 15 atomes de carbone, ou un groupe alcoxyalkyle inférieur dont le fragment alkyle comporte 8 à 15 atomes de carbone et le fragment alcoxy comporte 1 à 4 atomes de carbone ;
B) le groupe phénylalkyle, dans lequel le fragment alkyle comporte de 1 à 6 atomes de carbone et le fragment phényle n'est pas substitué ou est substitué et, dans ce cas, les substituants peuvent être fixés en position ortho, méta ou para du noyau phényle et ils sont des atomes de chlore, de fluor ou de brome, un groupe alkyle inférieur ayant 1 à 4 atomes de carbone, alcoxy inférieur ayant 1 à 4 atomes de carbone, hydroxy ou méthylènedioxy ;
C) le groupe

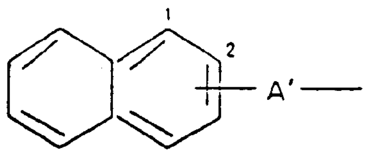

dans lequel A′ représente un groupe alkylène linéaire ou ramifié ayant 1 à 6 atomes de carbone ou un groupe benzyle, et il est fixé sur la position 1 ou 2 du noyau naphtalénique, qui n'est pas substitué ou est monosubstitué ou disubstitué par un atome de chlore, de fluor, de brome, un groupe trifluorométhyle, un groupe alkyle linéaire ou ramifié ayant 1 à 12 atomes de carbone, un groupe alcoxy ayant 1 à 3 atomes de carbone ou $NO_2$,

D) le groupe 1- ou 2-adamantyle ou 1- ou 2-norbornyle

E) le groupe ortho-, méta- ou para-biphénylyle ;

F) le groupe 9-fluorényle, qui n'est pas substitué ou est substitué et, dans ce cas, les substituants peuvent être fixés sur l'un quelconque des quatre atomes de carbone disponibles de chaque noyau aromatique et ce sont des atomes de chlore, de brome, de fluor, un groupe alkyle inférieur ayant 1 à 4 atomes de carbone, un groupe alcoxy inférieur ayant 1 à 4 atomes de carbone ou $NO_2$ ;

G) un groupe dibenzocycloheptényle

H) le groupe

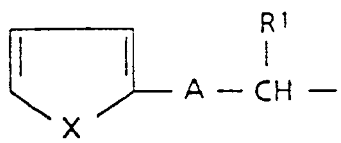

dans lequel X représente un atome d'oxygène ou de soufre ; A est une liaison ou représente une chaîne alkylène ayant 1 à 3 atomes de carbone, et $R^1$ représente un atome d'hydrogène, un groupe alkyle inférieur, linéaire ou ramifié, ayant 1 à 4 atomes de carbone, un groupe alcényle inférieur, linéaire ou ramifié, ayant de 3 à 6 atomes de carbone, cycloalkyle ayant de 3 à 6 atomes de carbone ou phényle ;

I) le groupe

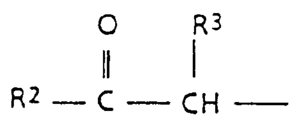

dans lequel $R^2$ représente un groupe phényle ou un groupe cycloalkyle ayant de 3 à 6 atomes de carbone, et $R^3$ représente un atome d'hydrogène ou un groupe méthyle ;

J) le groupe

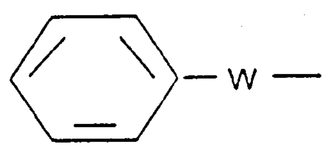

dans lequel W représente une chaîne alkylène linéaire ou ramifiée ayant de 2 à 6 atomes de carbone, qui est substituée par un groupe phényle fixé sur l'un quelconque des 6 atomes de carbone, à la condition que l'atome de carbone adjacent de l'azote exocyclique comporte au moins un atome d'hydrogène fixé sur lui ;

K) le groupe

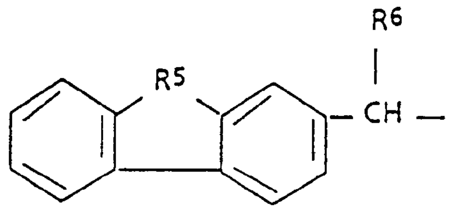

dans lequel $R^5$ représente un atome d'oxygène ou de soufre, un groupe $-CH_2CH_2-$ ou -CH=CH-, et $R^6$ représente un atome d'hydrogène ou un groupe alkyle inférieur ayant 1 à 4 atomes de carbone ;
L) Le groupe

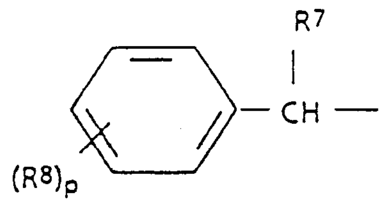

dans lequel $R^7$ représente un groupe cycloalkyle ayant de 3 à 5 atomes de carbone, $R^8$ représente un atome d'hydrogène, un groupe alcoxy inférieur ayant de 1 à 4 atomes de carbone ou un groupe alkyle inférieur ayant 1 à 4 atomes de carbone, et p est un nombre entier valant 1 ou 2 ;
M) le groupe

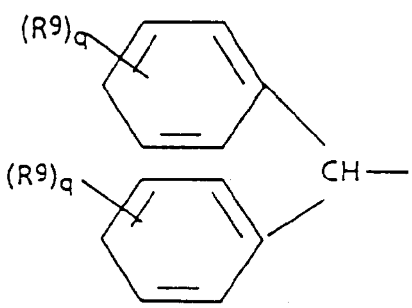

dans lequel $R^9$ représente un atome d'hydrogène, un groupe alkyle inférieur, linéaire ou ramifié, ayant 1 à 4 atomes de carbone, un atome de chlore, de fluor, de brome, un groupe $CF_3$, $SCF_3$, $OCF_3$, phényle, phénoxy ou un groupe alcoxy inférieur ayant 1 à 4 atomes de carbone, et q est un nombre entier valant 1 à 3 ;
N) le groupe

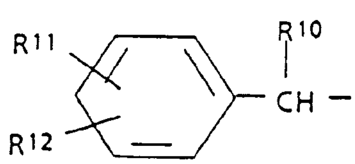

dans lequel $R^{10}$ représente un groupe alkyle inférieur ayant 1 à 4 atomes de carbone, $R^{11}$ représente un atome d'hydrogène ou un groupe alkyle inférieur ayant 1 à 4 atomes de carbone, $R^{12}$ représente un groupe alkyle ayant 8 à 14 atomes de carbone, un groupe alcoxy ayant 8 à 14 atomes de carbone, un groupe cycloalkyle ayant 5 à 14 atomes de carbone, un groupe phényle, phénoxy, phénylalkyle dans lequel le fragment alkyle comporte 1 à 4 atomes de carbone, phénylalcoxy dans lequel le fragment alcoxy comporte 2 à 4 atomes de carbone, un groupe 2,2-diphénylvinyle ou fluorène-9-ylidène ;
O) le groupe

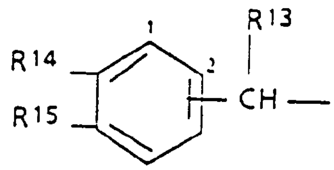

dans lequel le fragment phényle est fixé sur le fragment

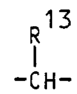

par la position 1 ou 2 du noyau phényle, $R^{13}$ représente un groupe alkyle inférieur ayant 1 à 4 atomes de carbone, et R 14 et R 15 pris ensemble, représentent $(-CH_2-)_3-CH_2CH_2C(CH_3)_2-$, $-(CH_2)_4-$ ou $-C(CH_3)_2CH_2CH_2C(CH_3)_2-$ ;

P) le groupe

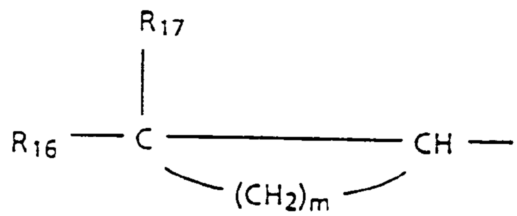

dans lequel $R^{16}$ représente un groupe thiényle, cycloalkyle ayant 5 à 7 atomes de carbone ou un groupe benzyle, phényle ou phényle ou benzyle substitué, et, dans ce cas, les substituants fixés sur le noyau ohényle substitué sont choisis parmi un atome de fluor, de chlore, de brome, d'iode, un groupe alkyle inférieur ayant 1 à 4 atomes de carbone et alcoxy inférieur ayant 1 à 4 atomes de carbone ;

Z représente un atome d'hydrogène ou un groupe hydroxy ; et m est un nombre entier valant 3 à 6 ;

à la condition que, lorsque R représente le groupe

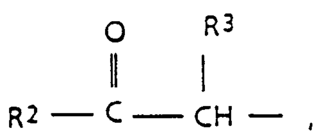

Z représente un atome d'hydrogène ;

et ses sels d'addition d'acide pharmaceutiquement acceptable ; en association avec un agent antiprotozoaire.

2. Composition selon la revendication 1, dans laquelle n représente le nombre entier 11.

3. Composition selon les revendications 1 ou 2, dans laquelle R représente un groupe 1- ou 2-adamantyle ou 1- ou 2-norbornyle.

4. Composition selon la revendication 3, dans laquelle R représente un groupe 2-norbornyle.

5. Composition selon la revendication 1, dans laquelle le composé est le 2-(2-norbornylimino)azacyclotridécane ou un sel pharmaceutiquement acceptable de ce composé.

6. Composition selon les revendications 1 ou 2, dans laquelle R représente

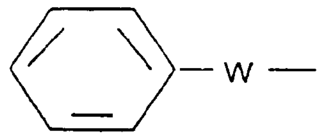

où
W représente une chaîne alkylène, linéaire ou ramifiée, ayant de 2 à 6 atomes de carbone, qui est substituée par un groupe phényle, 1- ou 2-adamantyle ou 1- ou 2-norbornyle sur l'un quelconque des 6 atomes de carbone, à la condition que l'atome de carbone adjacent à l'atome d'azote exocyclique comporte au moins un atome d'hydrogène qui lui est fixé.

7. Composition selon les revendications 1 ou 2, dans laquelle R représente un groupe 2,2-diphénylpentyle.

8. Composition selon les revendications 1 ou 2, dans laquelle R représente un groupe (α-(1-adamantyl)benzyle.

9. Composition selon les revendications 1 ou 2, dans laquelle R représente un groupe 3,3-diphénylpropyle.

10. Composition selon les revendications 1 ou 2 dans laquelle R représente un groupe ortho-, méta- ou parabiphénylyle.

11. Composition selon la revendication 10, dans laquelle R représente un groupe ortho-biphénylyle.

12. Composition selon la revendication 1, dans laquelle le composé est le 2-(o-biphénylylimino)azacyclotridécane ou un de ses sels pharmaceutiquement acceptables.

13. Composition selon la revendication 1, dans laquelle le composé est le 2-[(cis-2-phénylcyclopentyl)imino]azacyclotridécane ou un sel pharmaceutiquement acceptable de ce composé.

14. Composition selon l'une quelconque des revendications 1 à 13, dans laquelle l'agent antiprotozoaire est de la quinine, de la chloroquine, de l'amodiaquine, de la primaquine ou de la méfloquine.

15. Composition selon l'une quelconque des revendications 1 à 14, pour l'utilisation séparée, successivement ou en même temps, dans le traitement curatif ou préventif d'une infection due à des protozoaires résistant aux médicaments, l'agent antiprotozoaire étant présent en des quantités efficaces pour assurer un traitement curatif ou Préventif de l'infection qui résiste aux médicaments.

16. Composition selon la revendication 15, dans laquelle l'infection par des protozoaires résistant à des médicaments est une infection de malaria résistant aux médicaments.

17. Composition selon l'une quelconque des revendications 1 à 16, avec un excipient pharmaceutiquement acceptable.

**Revendications pour l'Etat contractant suivant : GR**

1. Utilisation séparée, successivement ou en même temps, d'un lactamimide, composé de formule I

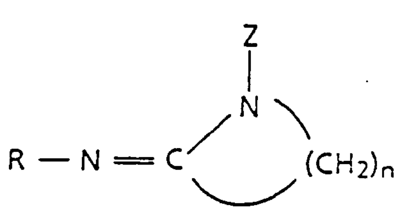

(I)

dans laquelle
Z représente un atome d'hydrogène ou un groupe alkyle inférieur ayant 1 à 4 atomes de carbone ;
n est un nombre entier valant 3 à 16 ;

R représente :

A) un groupe alkyle, linéaire ou ramifié, ayant 8 à 15 atomes de carbone, ou un groupe alcoxyalkyle inférieur dont le fragment alkyle comporte 8 à 15 atomes de carbone et le fragment alcoxy comporte 1 à 4 atomes de carbone ;

B) le groupe phénylalkyle, dans lequel le fragment alkyle comporte de 1 à 6 atomes de carbone et le fragment phényle n'est pas substitué ou est substitué et, dans ce cas les substituants peuvent être fixés en position ortho, méta ou para du noyau phényle et ils sont des atomes de chlore, de fluor ou de brome, un groupe alkyle inférieur ayant 1 à 4 atomes de carbone, alcoxy inférieur ayant 1 à 4 atomes de carbone, hydroxy au méthylènedioxy ;

C) le groupe

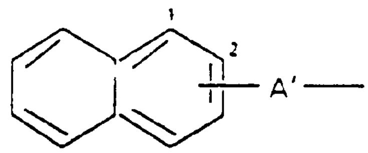

dans lequel A' représente un groupe alkylène linéaire ou ramifié ayant 1 à 6 atomes de carbone ou un groupe benzyle, et il est fixé sur la position 1 ou 2 du noyau naphtalénique, qui n'est pas substitué ou est monosubstitué ou disubstitué par un atome de chlore, de fluor, de brome, un groupe trifluorométhyle, un groupe alkyle linéaire ou ramifié ayant 1 à 12 atomes de carbone, un groupe alcoxy ayant 1 à 3 atomes de carbone ou $NO_2$ ;

D) le groupe 1- ou 2-adamantyle ou 1- ou 2-norbornyle

E) le groupe ortho-, méta- ou para-biphénylyle ;

F) le groupe 9-fluorényle, qui n'est pas substitué ou est substitué et, dans ce cas, les substituants peuvent être fixés sur l'un quelconque des quatre atomes de carbone disponibles de chaque noyau aromatique et ce sont des atomes de chlore, de brome, de fluor, un groupe alkyle inférieur ayant 1 à 4 atomes de carbone, un groupe alcoxy inférieur ayant 1 à 4 atomes de carbone ou $NO_2$ ;

G) un groupe dibenzocyclaheptényle

H) le groupe

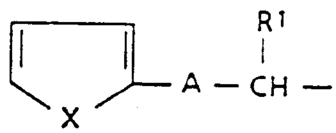

dans lequel X représente un atome d'oxygène ou de soufre ; A est une liaison ou représente une chaîne alkylène ayant 1 à 3 atomes de carbone, et $R^1$ représente un atome d'hydrogène, un groupe alkyle inférieur, linéaire ou ramifié, ayant 1 à 4 atomes de carbone, un groupe alcényle inférieur, linéaire ou ramifié, ayant de 3 à 6 atomes de carbone, cycloalkyle ayant de 3 à 6 atomes de carbone ou phényle ;

I) le groupe

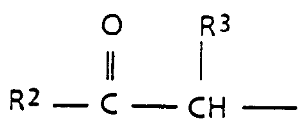

dans lequel $R^2$ représente un groupe phényle au un groupe cycloalkyle ayant de 3 à 6 atomes de carbone, et $R^3$ représente un atome d'hydrogène ou un groupe méthyle ;

J) le groupe

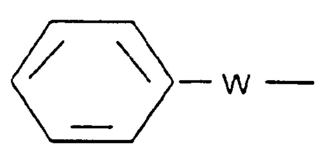

dans lequel W représente une chaîne alkylène linéaire ou ramifiée ayant de 2 à 6 atomes de carbone, qui est substituée par un groupe phényle fixé sur l'un quelconque des 6 atomes de carbone, à la condition que l'atome de carbone adjacent de l'azote exocyclique comporte au moins un atome d'hydrogène fixé sur lui ;

K) le groupe

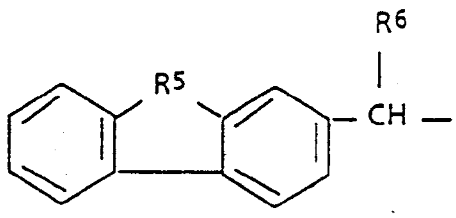

dans lequel $R^5$ représente un atome d'oxygène ou de soufre, un groupe $-CH_2CH_2-$ ou -CH=CH-, et $R^6$ représente un atome d'hydrogène ou un groupe alkyle inférieur ayant 1 à 4 atomes de carbone ;

L) le groupe

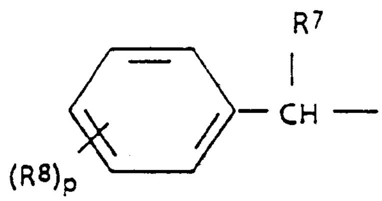

dans lequel $R^7$ représente un groupe cycloalkyle ayant de 3 à 5 atomes de carbone, $R^8$ représente un atome d'hydrogène, un groupe alcoxy inférieur ayant de 1 à 4 atomes de carbone ou un groupe alkyle inférieur ayant 1 à 4 atomes de carbone, et p est un nombre entier valant 1 ou 2 ;

M) le groupe

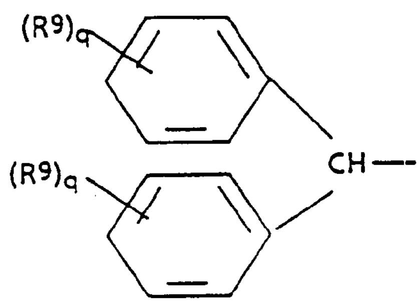

dans lequel $R^9$ représente un atome d'hydrogène, un groupe alkyle inférieur, linéaire ou ramifié, ayant 1 à 4 atomes de carbone, un atome de chlore, de fluor,. de brome, un groupe $CF_3$, $SCF_3$, $OCF_3$, phényle, phénoxy ou un groupe alcoxy inférieur ayant 1 à 4 atomes de carbane, et q est un nombre entier valant 1 à 3 ;

N) le groupe

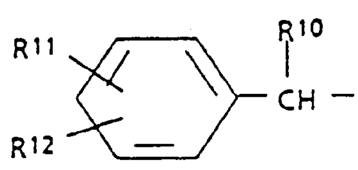

dans lequel $R^{10}$ représente un groupe alkyle inférieur ayant 1 à 4 atomes de carbone, R il représente un atome d'hydrogène ou un groupe alkyle inférieur ayant 1 à 4 atomes de carbone, $R^{12}$ représente un groupe alkyle ayant 8 à 14 atomes de carbone, un groupe alcoxy ayant 8 à 14 atomes de carbone, un groupe cycloalkyle ayant 5 à 14 atomes de carbone, un groupe phényle, phénoxy, phénylalkyle dans lequel le fragment alkyle comporte à 4 atomes de carbone, ohénylalcoxy dans lequel le fragment alcoxy comporte 2 à 4 atomes de carbone, un groupe 2,2-diphénylvinyle ou fluorène-9-ylidène ;
O) le groupe

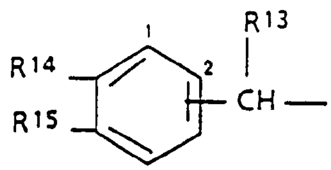

dans lequel le fragment phényle est fixé sur le fragment

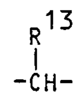

par la position 1 ou 2 du noyau phényle, $R^{13}$ représente un groupe alkyle inférieur ayant 1 à 4 atomes de carbane, et $R^{14}$ et $R^{15}$ pris ensemble, représentent $(-CH_2-)_3-CH_2CH_2C(CH_3)_2-$, $-(CH_2)_4-$ ou $-C(CH_3)_2CH_2CH_2C(CH_3)_2-$ ;
P) le groupe

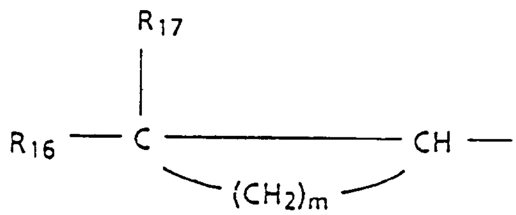

dans lequel $R^{16}$ représente un groupe thiényle, cycloalkyle ayant 5 à 7 atomes de carbone ou un groupe benzyle, ohényle ou phényle ou benzyle substitué, et, dans ce cas, les substituants fixés sur le noyau ohényle substitué sont choisis parmi un atome de fluor, de chlore, de brome, d'iode, un groupe alkyle inférieur ayant 1 à 4 atomes de carbone et alcoxy inférieur ayant 1 à 4 atomes de carbane ;
Z représente un atome d'hydrogène ou un groupe hydroxy ; et m est un nombre entier valant 3 à 6 ;
à la condition que, lorsque R représente le groupe

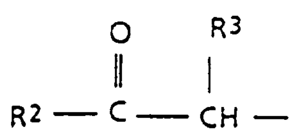,

Z représente un atome d'hydrogène ;
et ses sels d'addition d'acide pharmaceutiquement acceptable, en association avec un agent antiprotozoaire, pour la préparation d'une composition pharmaceutique.

2. Utilisation selon la revendication 1, dans laquelle n représente le nombre entier 11.

3. Utilisation selon les revendications 1 ou 2, dans laquelle R représente un groupe 1- ou 2-adamantyle ou 1- ou 2-norbornyle.

4. Utilisation selon la revendication 3, dans laquelle R représente un groupe 2-norbornyle.

5. Utilisation selon la revendication 1. dans laquelle le composé est le 2-(2-norbornylimino)azacyclotridéca-

ne ou un sel pharmaceutiquement acceptable de ce composé.

6. Utilisation selon la revendication 1 ou 2, dans laquelle R représente

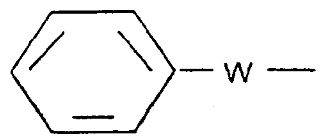

où
W est une chaîne alkylène, linéaire ou ramifiée, ayant 2 à 6 atomes de carbone, qui est substituée par un groupe phényle, 1- ou 2-adamantyle ou 1- ou 2-norbornyle sur l'un quelconque des 6 atomes de carbone, à la condition que l'atome de carbone voisin de l'atome d'azote exocyclique comporte bien au moins un atome d'hydrogène qui y est fixé.

7. Utilisation selon les revendications 1 ou 2, dans laquelle R représente un groupe 2,2-diphénylpentyle.

8. Utilisation selon les revendications 1 ou 2, dans laquelle R représente un groupe (α-(1-adamantyl)benzyle.

9. Utilisation selon les revendications 1 ou 2, dans laquelle R représente un groupe 3,3-diphénypropyle.

10. Utilisation selon les revendications 1 ou 2, dans laquelle R représente un groupe ortho-, méta- ou para-biphénynyle.

11. Utilisation selon la revendication 10, dans laquelle R représente un groupe ortho-bisphénylyle.

12. Utilisation selon les revendications 1 ou 2, dans laquelle le composé est le 2-(o-biphénylylimino)azacyclotridécane ou un sel pharmaceutiquement acceptable de ce composé.

13. Utilisation selon la revendication 1, dans laquelle le composé est le 2-[(cis-2-phénylcyclopentyl)imino]azacyclotridécane ou un de ses sels pharmaceutiquement acceptables.

14. Utilisation selon l'une quelconque des revendications 1 à 13, dans laquelle l'agent antiprotozoaire est de la quinine, de la chloroquine, de l'amodiaquine, de la primaquine ou de la méfloquine.

15. Utilisation selon l'une quelconque des revendications 1 à 14, dans laquelle la composition pharmaceutique est une composition pour le traitement curatif ou préventif d'une infection, résistante aux médicaments, et l'agent antiprotozoaire est ajouté en des quantités efficaces pour traiter l'infection qui résiste aux médicaments.

16. Utilisation selon la revendication 15, dans laquelle l'infection par des protozoaires résistant à des médicaments est une infection de malaria résistant aux médicaments.

17. Utilisation selon l'une quelconque des revendications 1 à 16, dans laquelle la composition contient en outre un excipient pharmaceutiquement acceptable.

**Revendications pour l'Etat contractant suivant : ES**

1. Procédé pour la préparation d'une composition, comprenant le mélangeage d'un lactamimide de formule I

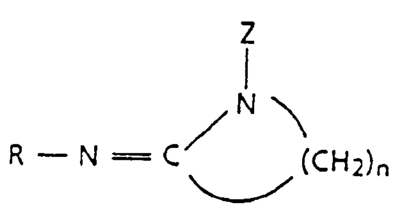

(I)

dans laquelle

Z représente un atome d'hydrogène ou un groupe alkyle inférieur ayant 1 à 4 atomes de carbone ;

n est un nombre entier valant 3 à 16 ;

R représente :

A) un groupe alkyle, linéaire ou ramifié, ayant 8 à 15 atomes de carbone, ou un groupe alcoxyalkyle inférieur dont le fragment alkyle comporte 8 à 15 atomes de carbone et le fragment alcoxy comporte 1 à 4 atomes de carbone ;

B) le groupe phénylalkyle, dans lequel le fragment alkyle comporte de 1 à 6 atomes de carbone et le fragment phényle n'est pas substitué ou est substitué et, dans ce cas, les substituants peuvent être fixés en position ortho, méta ou para du noyau phényle et ils sont des atomes de chlore, de fluor ou de brome, un groupe alkyle inférieur ayant 1 à 4 atomes de carbone, alcoxy inférieur ayant 1 à 4 atomes de carbone, hydroxy ou méthylènedioxy ;

C) le groupe

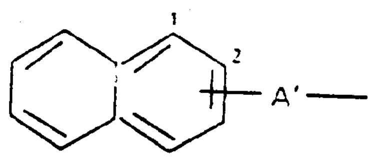

dans lequel A′ représente un groupe alkylène linéaire ou ramifié ayant 1 à 6 atomes de carbone ou un groupe benzyle, et il est fixé sur la position 1 ou 2 du noyau naphtalénique, qui n'est pas substitué au est monosubstitué ou disubstitué par un atome de chlore, de fluor, de brome, un groupe trifluorométhyle, un groupe alkyle linéaire ou ramifié ayant 1 à 12 atomes de carbone, un groupe alcoxy ayant 1 à 3 atomes de carbone ou $NO_2$ ;

D) le groupe 1- ou 2-adamantyle ou 1- ou 2-norbornyle

E) le groupe ortho-, méta- ou para-biphénylyle ;

F) le groupe 9-fluorényle, qui n'est pas substitué ou est substitué et, dans ce cas, les substituants Peuvent être fixés sur l'un quelconque des quatre atomes de carbone disponibles de chaque noyau aromatique et ce sont des atomes de chlore, de brome, de fluor, un groupe alkyle inférieur ayant 1 à 4 atomes de carbone, un groupe alcoxy inférieur ayant 1 à 4 atomes de carbone ou $NO_2$ ;

G) un groupe dibenzocycloheptényle ;

H) le groupe

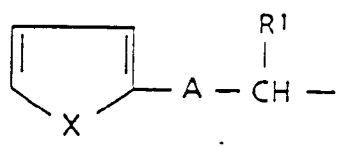

dans lequel X représente un atome d'oxygène ou de soufre ; A est une liaison ou représente une chaîne alkylène ayant 1 à 3 atomes de carbone, et $R^1$ représente un atome d'hydrogène, un groupe alkyle inférieur, linéaire ou ramifié, ayant 1 à 4 atomes de carbone, un groupe alcényle inférieur, linéaire ou ramifié, ayant de 3 à 6 atomes de carbone, cycloalkyle ayant de 3 à 6 atomes de carbone ou phényle ;

I) le groupe

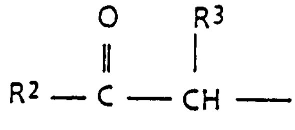

dans lequel $R^2$ représente un groupe phényle ou un groupe cycloalkyle ayant de 3 à 6 atomes de carbone, et $R^3$ représente un atome d'hydrogène ou un groupe méthyle ;

J) le groupe

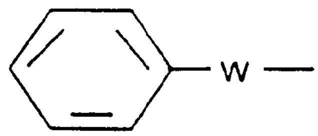

dans lequel W représente une chaîne alkylène linéaire ou ramifiée ayant de 2 à 6 atomes de carbone, qui est substituée par un groupe phényle fixé sur l'un quelconque des 6 atomes de carbone, à la condition que l'atome de carbone adjacent de l'azote exocyclique comporte au moins un atome d'hydrogène fixé sur lui ;

K) le groupe

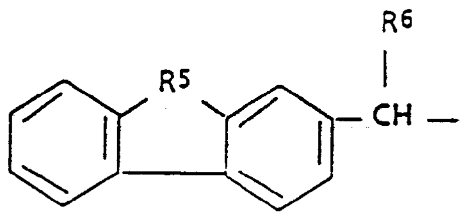

dans lequel $R^5$ représente un atome d'oxygène ou de soufre, un groupe $-CH_2CH_2-$ ou -CH=CH-, et $R^6$ représente un atome d'hydrogène ou un groupe alkyle inférieur ayant 1 à 4 atomes de carbone ;

L) le groupe

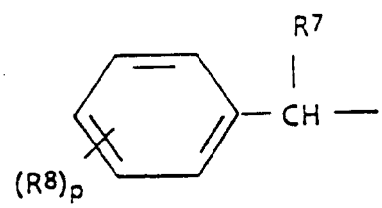

dans lequel $R^7$ représente un groupe cycloalkyle ayant de 3 à 5 atomes de carbone, $R^8$ représente un atome d'hydrogène, un groupe alcoxy inférieur ayant de 1 à 4 atomes de carbone ou un groupe alkyle inférieur ayant 1 à 4 atomes de carbone, et p est un nombre entier valant 1 ou 2 ;

M) le groupe

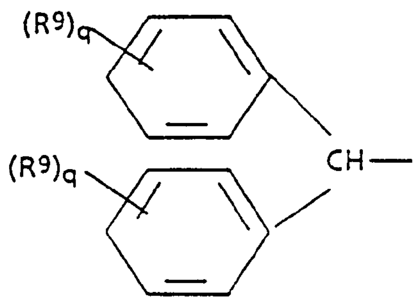

dans lequel $R^9$ représente un atome d'hydrogène, un groupe alkyle inférieur, linéaire ou ramifié, ayant 1 à 4 atomes de carbone, un atome de chlore, de fluor, de brome, un groupe $CF_3$, $SCF_3$, $OCF_3$, phényle, phénoxy ou un groupe alcoxy inférieur ayant 1 à 4 atomes de carbone, et q est un nombre entier valant 1 à 3 ;

N) le groupe

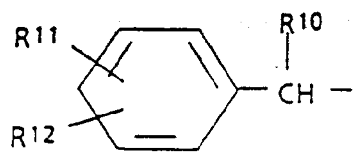

dans lequel $R^{10}$ représente un groupe alkyle inférieur ayant 1 à 4 atomes de carbone, $R^{11}$ représente un atome d'hydrogène ou un groupe alkyle inférieur ayant 1 à 4 atomes de carbone, R 12 représente un groupe alkyle ayant 8 à 14 atomes de carbone, un groupe alcoxy ayant 8 à 14 atomes de carbone, un groupe cycloalkyle ayant 5 à 14 atomes de carbone, un groupe phényle, phénoxy, phénylalkyle dans lequel le fragment alkyle comporte 1 à 4 atomes de carbone, ohénylalcoxy dans lequel le fragment alcoxy comporte 2 à 4 atomes de carbone, un groupe 2,2-diphénylvinyle ou fluorène-9-ylidène ;

O) le groupe

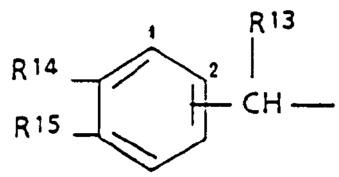

dans lequel le fragment phényle est fixé sur le fragment

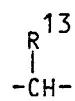

par la position 1 ou 2 du noyau phényle, $R^{13}$ représente un groupe alkyle inférieur ayant 1 à 4 atomes de carbone, et $R^{14}$ et $R^{15}$ pris ensemble, représentent $(-CH_2-)_3-CH_2CH_2C(CH_3)_2-$, $-(CH_2)_4-$ ou $-C(CH_3)_2CH_2CH_2C(CH_3)_2-$ ;

P) le groupe

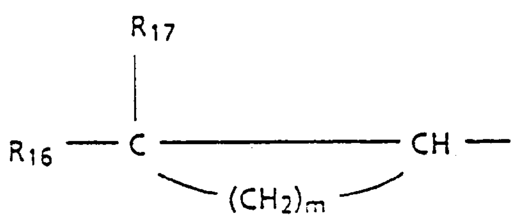

dans lequel $R^{16}$ représente un groupe thiényle, cycloalkyle ayant 5 à 7 atomes de carbone ou un groupe benzyle, phényle ou phényle ou benzyle substitué, et, dans ce cas, les substituants fixés sur le noyau phényle substitué sont choisis parmi un atome de fluor, de chlore, de brome, d'iode, un groupe alkyle inférieur ayant 1 à 4 atomes de carbone et alcoxy inférieur ayant 1 à 4 atomes de carbone ; Z représente un atome d'hydrogène ou un groupe hydroxy ; et m est un nombre entier valant 3 à 6 ;

à la condition que, lorsque R représente le groupe

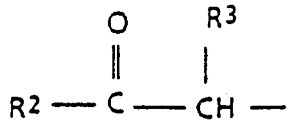 ,

Z représente un atome d'hydrogène ;

et ses sels d'addition d'acide pharmaceutiquement acceptable ; avec un agent antiprotozoaire.

2. Procédé selon la revendication 1, dans lequel n représente le nombre entier 11.

3. Procédé selon les revendications 1 ou 2, dans lequel R représente un groupe 1- au 2-adamantyle ou 1- ou 2-norbornyle.

4. Procédé selon la revendication 3, dans lequel R représente un groupe 2-norbornyle.

5. Procédé selon la revendication 1, dans lequel le composé est le 2-(2-norbornylimino)azacyclotridécane ou un de ses sels pharmaceutiquement acceptables.

6. Procédé selon les revendications 1 ou 2, dans lequel R représente

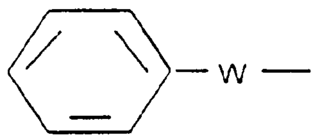

où
W représente une chaîne alkylène linéaire ou ramifiée ayant de 2 à 6 atomes de carbone qui est substituée par un groupe phényle, 1-ou 2-adamantyle, ou 1- ou 2-norbornyle sur l'un quelconque des 6 atomes de carbone, à la condition que l'atome de carbone voisin de l'atome d'azote exocyclique comporte bien au moins un atome d'hydrogène qui y est fixé.

7. Procédé selon les revendications 1 ou 2, dans lequel R représente un groupe 2,2-diphénylpentyle.

8. Procédé selon les revendications 1 ou 2, dans lequel R représente un groupe α-(1-adamantyl)benzyle.

9. Procédé selon les revendications 1 ou 2, dans lequel R représente un groupe 3,3-diphénylpropyle.

10. Procédé selon les revendications 1 ou 2, dans lequel R représente un groupe ortho-, méta- ou para-biphénylyle.

11. Procédé selon la revendication 10, dans lequel R représente un groupe ortho-biphényle.

12. Procédé selon les revendications 1 ou 2, dans lequel le composé est le 2-(o-biphénylylimino)azacyclotridécane ou un de ses sels pharmaceutiquement acceptables.

13. Procédé selon la revendication 1, dans lequel le composé est le 2-[(cis-2-phénylcyclopentyl)imino]azacyclotridécane ou un de ses sels pharmaceutiquement acceptables.

14. Procédé selon l'une quelconque des revendications 1 à 13, dans lequel l'agent antiprotozoaire est de la quinine, de la chloroquine, de l'amodiaquine, de la primaquine ou de la méfloquine.

15. Procédé selon l'une quelconque des revendications 1 à 14, dans lequel la composition contient en outre un excipient ou véhicule pharmaceutiquement acceptable.

SYNERGISM BETWEEN CHLOROQUINE AND
2-[(CIS-2-PHENYLCYCLOPENTYL)IMINO]AZACYCLOTRIDECANE HYDROCHLORIDE
AGAINST A CHLOROQUINE-RESISTANT PLASMODIUM FALCIPARUM (FCR-3)

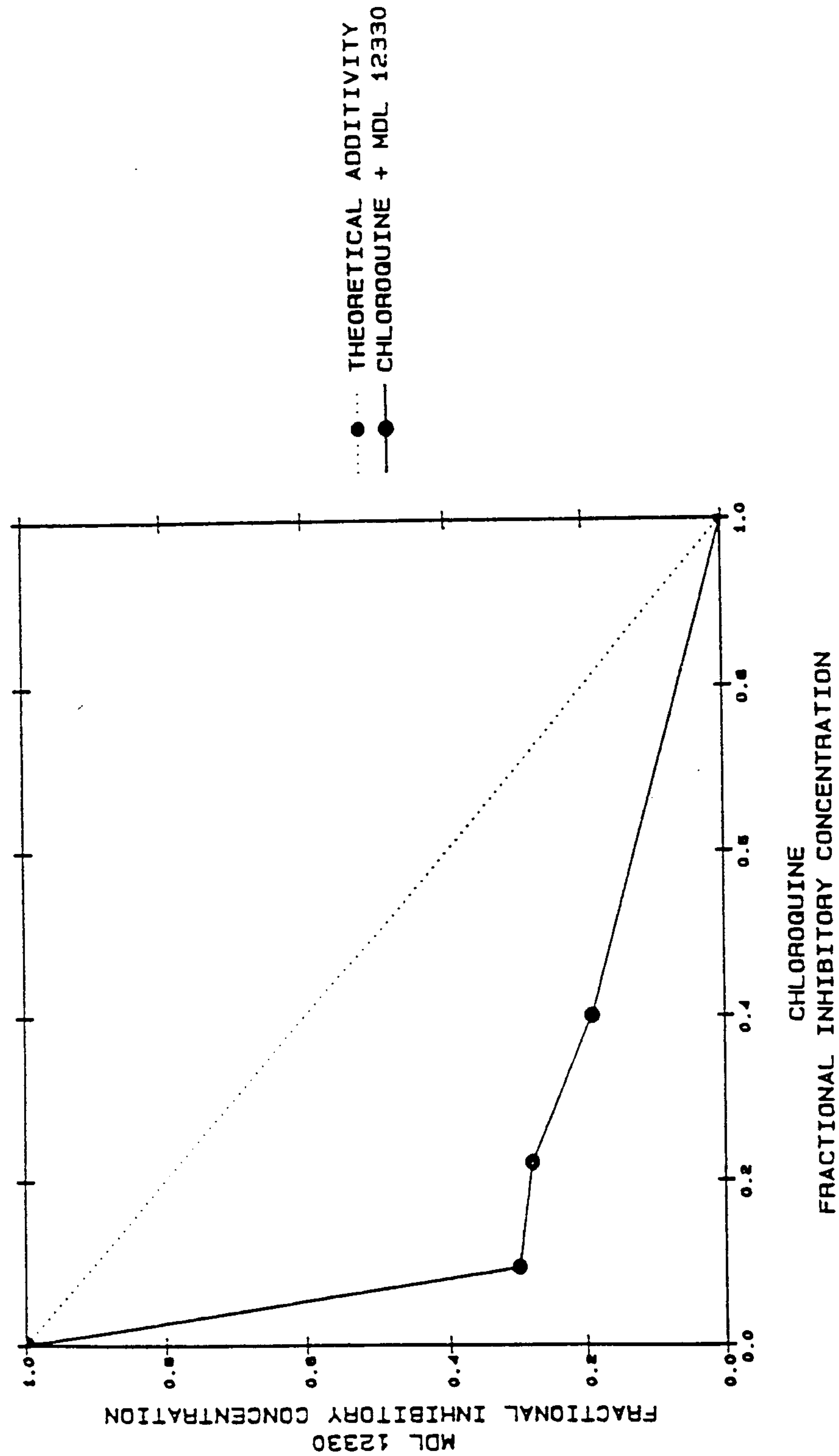


FIGURE 1